# EUROPEAN PATENT APPLICATION

(11) **EP 4 822 020 A1**
(43) Date of publication of application: **14.10.2026**
(21) Application number: 24899940.1
(22) Date of filing: 06.12.2024
(51) Int. Cl.: C12N 15/09, A61K 39/00, A61K 35/17

(54) **BASE EDITING SYSTEM FOR OPTIMIZING ENGINEERED T-CELLS, AND USE THEREOF**

(30) Priority: 06.12.2023 CN 202311662136; 11.01.2024 CN 202410044584; 04.06.2024 CN 202410718712
(71) Applicant: Beijing Qi Biodesign Biotechnology Company Limited, Beijing 102209 (CN)
(72) Inventor: ZHAO, Kevin T., Beijing 102206 (CN); HU, Jiacheng, Beijing 102206 (CN)
(74) Representative: Weickmann & Weickmann PartmbB
(86) International application number: PCT/CN2024/137280
(87) International publication number: WO 2025/119306

(57) **Abstract**

A base editing system for optimizing engineered T-cells, and a use thereof. Provided is a base editing system for modifying engineered T-cells, comprising: i) a base editing polypeptide, and/or an expression construct encoding the base editing polypeptide, the base editing polypeptide comprising a DNA binding protein and at least one deaminase domain; and ii) a guide RNA, and/or an expression construct encoding the guide RNA, the guide RNA targeting any one or any combination of the TRAC, TGFBR2, PD-1, CISH and/or FAS genome regions. The base editing system has efficient gene editing tool efficiency and low indel frequency, can implement precise editing of genes relating to T-cell function enhancement, and has important value for expanding application scenarios of base editing technology in fields such as the preparation of safe and efficient engineered T-cells.

## Description

### CROSS-REFERENCE TO RELATED APPLICATIONS

The present application claims the benefits of priority of the Chinese Patent Application No. 202311662136.4, filed on December 6, 2023 and entitled "OPTIMIZED BASE EDITING SYSTEM AND USE THEREOF", the Chinese Patent Application No. 202410044584.6, filed on January 11, 2024 and entitled "OPTIMIZED BASE EDITING SYSTEM AND USE THEREOF", and the Chinese Patent Application No. 202410718712.0, filed on June 4, 2024 and entitled "OPTIMIZED BASE EDITING SYSTEM AND USE THEREOF", the entire contents of which including the appendices are incorporated herein by reference.

### TECHNICAL FIELD

The present disclosure belongs to the field of genetic engineering. Specifically, the present disclosure relates to a base editing system for optimizing engineered T-cells, and use thereof, and more specifically to a base editing system and method for modifying or editing engineered T-cells.

### BACKGROUND

Chimeric Antigen Receptor T-cell (simply referred to as CAR-T-cell or CAR-T) is a type of T-cell modified by the genetic engineering technology. By integrating a recognition receptor directed against a specific target antigen epitope (*e.g.*, some tumor cell-specific antigen molecules) into the T lymphocyte genome and stimulating activation of the T-cells, the ability of T lymphocytes to recognize and kill a specific tumor cell is thereby enhanced.

Based on the sources of the modified T-cells, CAR-T-cells can be divided into two major categories: autologous CAR-T-cells and allogeneic (or off-the-shelf) CAR-T-cells. The former is obtained by extracting and modifying T-cells from patients themselves, whereas the latter is obtained by isolating and modifying T-cells from healthy allogeneic donors. Both types of CAR-T-cells have their respective advantages and disadvantages in practical applications. For example, a major advantage of autologous CAR-T-cells is that, because of originating from the patient's own body, they generally exhibit a better immune effect and do not cause immune rejection upon reinfusion into the patient after the *in vitro* CAR gene integration and modification. However, because the number of healthy T-cells is relatively limited in patients with tumor cells (particularly at the advanced stages of disease), it is sometimes difficult to isolate sufficient T-cells and complete preparation of autologous CAR-T-cells in a timely manner. In contrast, off-the-shelf CAR-T-cells can be isolated from healthy donors, ensuring a reliable cell supply, a shorter supply cycle, and a significant lower cost for patients. Thus, the off-the-shelf CAR-T-cells represent a highly promising novel CAR-T-cell therapy. Nonetheless, conventional off-the-shelf CAR-T-cells suffer from poor clinical efficacy, which is primarily attributable to two factors. First, because of originating from different individuals, the off-the-shelf CAR-T-cells may be subject to immune rejection upon infusion into the patient, leading to their rapid clearance by the patient's immune system. Second, the occurrence of graft-versus-host disease (GvHD) causes CAR-T-cells from allogeneic individuals to recognize and mistakenly attack the patient's own immune cells, thereby compromising the patient's immune system. The combination of these two factors has resulted in the poor clinical efficacy of the conventional off-the-shelf CAR-T-cells.

Recent studies have shown, however, that by knocking out certain genes via gene editing during the functional modifications and manufacturing of off-the-shelf CAR-T-cells, it is possible to not only suppress immune rejection and the occurrence of GvHD, but also help enhance the proliferation capacity, tumor-killing capacity and persistence of the off-the-shelf CAR-T-cells after infusion into patients, thereby improving their clinical efficacy.

For example, the *CISH* gene encodes an immune negative regulator that can suppress the IL-15 signaling pathway in NK cells and T-cells to inhibit immune responses. Studies have shown that downregulating the *CISH* gene expression contributes to activation, survival, and proliferation of the T-cells (Zhu et al., 2022. Metabolic Reprograming via Deletion of CISH in Human iPSC-Derived NK Cells Promotes In Vivo Persistence and Enhances Anti-tumor Activity.).

FAS encoded by the *FAS* gene is a receptor protein that interacts with the FASL protein to promote T-cell apoptosis. Studies have shown that by expressing a dominant-negative effector protein of the FAS protein in the CAR-T-cells to disrupt the interaction between FAS and FASL, it is possible to improve the persistence of the CAR-T-cells and enhance their tumor-killing efficacy (Yamamoto et al., 2019. T-cells genetically engineered to overcome death signaling enhance adoptive cancer immunotherapy).

TGF-β is an anti-inflammatory cytokine expressed in the blood and in the immune microenvironment of many tumors. Studies have shown that binding of TGF-β to the receptor protein TBR2 encoded by the *TGFBR2* gene can trigger activation of downstream signaling pathways that suppress T-cell activation and survival, thereby inhibiting T-cell immunity (Rouce et al., 2015. The TGF-β/SMAD pathway is an important mechanism for NK cell immune evasion in childhood B-acute lymphoblastic leukemia). Conversely, disruption of the binding and interaction between TGF-β and the receptor TBR2 can help enhance the activation and survival of the T-cells (Bollard et al., 2018. Tumor-Specific T-Cells Engineered to Overcome Tumor Immune Evasion Induce Clinical Responses in Patients With Relapsed Hodgkin Lymphoma). Therefore, knocking out the *TGFBR2* gene in an appropriate region may contribute to the application efficacy of the CAR-T-cells.

Furthermore, knockout of the immune checkpoint protein PD-1 has also been reported to promote the activation, proliferation, and tumor-killing effect of T-cells (McGowan et al., 2020. PD-1 disrupted CAR-T-cells in the treatment of solid tumors: Promises and challenges). Knockout of TRAC helps eliminate the GvHD reaction and improve the survival and tumor-killing effect of the CAR-T-cells (Eyquem et al., 2017. Targeting a CAR to the TRAC locus with CRISPR/Cas9 enhances tumour rejection).

Although knockout of one or several of these genes has been reported to improve the application efficacy of the CAR-T-cells, simultaneous knockout of the above five genes using base editing has not yet been reported.

Base editing refers to a process of nucleotide substitution at a specific DNA site by means of genetic engineering. Base editing is a type of genome editing technology. In humans and animals, many genetic diseases are caused by point mutations in functional genes. Base editing technology enables targeted modifications and engineering of genomic DNA in plants, animals, and microorganisms to create new genotypes and acquire target traits beneficial for production and application. It can also be used to correct some severe congenital genetic variations in the treatment of genetic diseases. Thus, base editing holds significant application prospects in the creation of elite germplasm and in the disease treatment.

Base editing is achieved by virtue of a base editor. According to the types of base on which the base editors act, the base editors can be classified into two categories: 1) a cytosine base editor (CBE) and 2) an adenine base editor (ABE). Of these, the CBE can act on the cytidine base of cytosine deoxyribonucleotides (simply referred to as cytidine, C), and the ABE can act on the adenosine base of adenine deoxyribonucleotides (simply referred to as adenosine, A). The CBE can convert the C-G base pair on the double-stranded DNA to the T-A base pair; and the ABE can convert the A-T base pair on the double-stranded DNA to the G-C base pair.

Currently, there still remains a need for precise, effective, stable, and safe base editing systems to further optimize the CAR-T-cells and improve the survival, proliferation, persistence, activation, and tumor-killing ability of the CAR-T-cells, while effectively suppressing or even eliminating the immune rejection or the GvHD reaction.

### SUMMARY

### Technical Problems

In view of the current need for precise, effective, stable, and safe base editing systems to further optimize cells, an objective of the present disclosure is to provide a base editing system capable of editing the genes associated with enhancement of T-cells to meet the practical requirements for optimizing engineered T-cells (*e.g.*, CAR-T-cells).

### Solutions to Problems

In the first aspect of the present disclosure, there is provided a method for obtaining a modified T-cell, wherein the method comprises knocking out or downregulating expression of an endogenous gene in a T-cell by a gene editing technology, wherein the endogenous gene comprises a T-cell receptor alpha chain constant (TRAC) gene, a transforming growth factor beta receptor 2 (TGFBR2) gene, a programmed death receptor 1 (PD-1) gene, a cytokine-inducible SH2-containing protein (CISH) gene, and a cell suicide-associated factor (FAS) gene.

In some embodiments, the gene editing technology comprises DNA insertion, deletion or substitution at a gene target, and preferably, the gene editing technology is DNA substitution at a gene target.

In some embodiments, the gene editing technology comprises single-base editing.

In some embodiments, the method comprises:
a) providing a purified T-cell population;
b) introducing a single-base editing system into the T-cell population of a); and
c) amplifying and harvesting the modified T-cell;
   wherein the single-base editing system in step b) comprises: i) a base editing polypeptide, and/or an expression construct encoding the base editing polypeptide; and ii) a guide RNA (gRNA) containing a spacer corresponding to a protospacer in the *TRAC*, *TGFBR2*, *PD-1*, *CISH* or *FAS* gene, and/or an expression construct encoding the guide RNA.

In some embodiments, a protospacer sequence of the *TRAC* gene comprises SEQ ID NO: 51; a protospacer sequence of the *TGFBR2* gene comprises SEQ ID NO: 21, SEQ ID NO: 24, SEQ ID NO: 27, or SEQ ID NO: 28; a protospacer sequence of the *PD-1* gene comprises SEQ ID NO: 41, SEQ ID NO: 44, SEQ ID NO: 45, SEQ ID NO: 46, or SEQ ID NO: 47; a protospacer sequence of the *CISH* gene comprises SEQ ID NO: 1, SEQ ID NO: 5, SEQ ID NO: 8, or SEQ ID NO: 10; and a protospacer sequence of the *FAS* gene comprises SEQ ID NO: 14, SEQ ID NO: 17, SEQ ID NO: 18, or SEQ ID NO: 20.

In some embodiments, a protospacer sequence of the *TRAC* gene comprises SEQ ID NO: 51; a protospacer sequence of the *TGFBR2* gene comprises SEQ ID NO: 24 or SEQ ID NO: 28; a protospacer sequence of the *PD-1* gene comprises SEQ ID NO: 41 or SEQ ID NO: 47; a protospacer sequence of the *CISH* gene comprises SEQ ID NO: 1; and a protospacer sequence of the *FAS* gene comprises SEQ ID NO: 14.

In some embodiments, the base editing polypeptide comprises a DNA-binding protein and at least one deaminase domain.

In some embodiments, the base editing polypeptide further comprises at least one uracil DNA glycosylase inhibitor (UGI) domain.

In some embodiments, the base editing polypeptide further comprises a nuclear localization sequence.

In some embodiments, the deaminase is a cytidine deaminase; optionally, the cytidine deaminase is an SCP1.201 family deaminase; alternatively, the SCP1.201 family deaminase is an Sdd2 deaminase, an Sdd3 deaminase, an Sdd4 deaminase, an Sdd6 deaminase, an Sdd7 deaminase, a mini-Sdd7 deaminase, a mini-Sdd9 deaminase, an Sdd10 deaminase, an Sdd59 deaminase, a mini-Sdd3 deaminase, or a mini-Sdd6 deaminase.

In some embodiments, the deaminase is Sdd7 and/or mini-Sdd9 cytidine deaminase(s).

In some embodiments, the DNA-binding protein is a TALE, ZFP or CRISPR effector protein or a variant thereof; optionally, the CRISPR effector protein is a Cas9 nickase, a deactivated Cas9, or a variant thereof.

In some embodiments, the base editing polypeptide comprises an amino acid sequence having at least 80%, 85%, 90%, 95%, 96%, 97%, 98%, or 99% sequence identity to any one of SEQ ID NOs: 74-80.

In some embodiments, the method further comprises introducing a nucleic acid sequence encoding a chimeric antigen receptor (CAR), wherein the chimeric antigen receptor (CAR) comprises an antigen-binding domain and a transmembrane domain.

In some embodiments, the T-cell population is T-cells derived from humans, optionally T-cells enriched from human blood cells, and alternatively cryopreserved T-cells enriched from human blood cells.

In some embodiments, a method for the introduction comprises electroporation, liposome transfection, microinjection, viral infection (such as adenovirus, adeno-associated virus, lentivirus, or other viruses), N-acetylgalactosamine (GalNAc)-mediated transformation, gene gun-mediated transformation, ribonucleoprotein (RNP) complex-mediated transformation, and nanoparticle-mediated transformation.

In the second aspect of the present disclosure, there is provided a base editing system of modifying a T-cell, comprising:
i) a base editing polypeptide, and/or an expression construct encoding the base editing polypeptide, wherein the base editing polypeptide comprises a DNA-binding protein and at least one deaminase domain; and
ii) a guide RNA, and/or an expression construct encoding the guide RNA, wherein the guide RNA targets any one or any combination of a T-cell receptor alpha chain constant (TRAC) gene, a transforming growth factor beta receptor 2 (TGFBR2) gene, a programmed death receptor 1 (PD-1) gene, a cytokine-inducible SH2-containing protein (CISH) gene, and/or a cell suicide-associated factor (FAS) genomic region.

In some embodiments, the deaminase domain is a cytidine deaminase.

In some embodiments, the cytidine deaminase is an APOBEC family deaminase, an SCP1.201 family deaminase or a homolog thereof;
optionally, the APOBEC family deaminase is an AID deaminase, an APOBEC1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, or an APOBEC3H deaminase; and
optionally, the SCP1.201 family deaminase is an Sdd2 deaminase, an Sdd3 deaminase, an Sdd4 deaminase, an Sdd6 deaminase, an Sdd7 deaminase, a mini-Sdd7 deaminase, a mini-Sdd9 deaminase, an Sdd10 deaminase, an Sdd59 deaminase, a mini-Sdd3 deaminase, or a mini-Sdd6 deaminase.

In some embodiments, the cytidine deaminase is Sdd7 and/or mini-Sdd9 cytidine deaminase(s).

In some embodiments, the DNA-binding protein is a TALE, ZFP or CRISPR effector protein.

In some embodiments, the CRISPR effector protein is Cas3, Cas4, Cas5, Cas5e (or CasD), Cas6, Cas6e, Cas6f, Cas7, Cas8a1, Cas8a2, Cas8b, Cas8c, Cas9, Cas10, Cast10d, Cas12, Cas13, Cas14, CasX, CasF, CasG, CasH, Csy1, Csy2, Csy3, Cse1 (or CasA), Cse2 (or CasB), Cse3 (or CasE), Cse4 (or CasC), Csc1, Csc2, Csa5, Csn1, Csn2, Csm2, Csm3, Csm4, Csm5, Csm6, Cmr1, Cmr3, Cmr4, Cmr5, Cmr6, Cpf1, Csb1, Csb2, Csb3, Csx17, Csx14, Csx10, Csx16, CsaX, Csx3, Csz1, Csx15, Csf1, Csf2, Csf3, Csf4, Cu1966, TraC, or a variant thereof.

In some embodiments, the DNA-binding protein is a Cas9 nickase, a deactivated Cas9 or a variant thereof.

In some embodiments, the base editing system further contains at least one uracil DNA glycosylase inhibitor (UGI).

In some embodiments, the base editing system further comprises a nuclear localization sequence.

In some embodiments, the base editing polypeptide comprises an amino acid sequence having at least 80% sequence identity to any one of SEQ ID NOs: 74-80.

In some embodiments, the base editing polypeptide comprises an amino acid sequence having at least 80%, 85%, 90%, 95%, 96%, 97%, 98%, or 99% sequence identity to any one of SEQ ID NOs: 77-80.

In some embodiments, the guide RNA contains a spacer corresponding to a protospacer in the *TRAC*, *TGFBR2*, *PD-1*, *CISH*, or *FAS* gene;
optionally, a protospacer sequence of the *TRAC* gene comprises SEQ ID NO: 51; a protospacer sequence of the *TGFBR2* gene comprises SEQ ID NO: 21, SEQ ID NO: 24, SEQ ID NO: 27, or SEQ ID NO: 28; a protospacer sequence of the *PD-1* gene comprises SEQ ID NO: 41, SEQ ID NO: 44, SEQ ID NO: 45, SEQ ID NO: 46, or SEQ ID NO: 47; a protospacer sequence of the *CISH* gene comprises SEQ ID NO: 1, SEQ ID NO: 5, SEQ ID NO: 8, or SEQ ID NO: 10; and a protospacer sequence of the *FAS* gene comprises SEQ ID NO: 14, SEQ ID NO: 17, SEQ ID NO: 18, or SEQ ID NO: 20.

In some embodiments, the protospacer sequence of the *TRAC* gene comprises SEQ ID NO: 51; the protospacer sequence of the *TGFBR2* gene comprises SEQ ID NO: 24 or SEQ ID NO: 28; the protospacer sequence of the *PD-1* gene comprises SEQ ID NO: 41 or SEQ ID NO: 47; the protospacer sequence of the *CISH* gene comprises SEQ ID NO: 1; and the protospacer sequence of the *FAS* gene comprises SEQ ID NO: 14.

In the third aspect of the present disclosure, there is provided a modified T-cell, wherein the T-cell is modified by the method according to the first aspect of the present disclosure, or introduced with the base editing system according to the second aspect of the present disclosure, and the T-cell has an endogenous gene knocked out or downregulated in expression, wherein the endogenous gene comprises a T-cell receptor alpha chain constant (TRAC) gene, a transforming growth factor beta receptor 2 (TGFBR2) gene, a programmed death receptor 1 (PD-1) gene, a cytokine-inducible SH2-containing protein (CISH) gene, and a cell suicide-associated factor (FAS) gene.

In some embodiments, the T-cell is a CAR-T-cell.

In some embodiments, the CAR-T-cell recognizes one or more target antigens.

In some embodiments, the T-cell is used for preventing or treating an autoimmune disease, a tumor, a viral infectious disease, a bacterial infectious disease, a genetic disease, an infectious disease, or a cardiovascular disease.

In the fourth aspect of the present disclosure, there is provided a composition, wherein the composition comprises the modified T-cell according to the third aspect of the present disclosure, and optionally a pharmaceutically acceptable carrier or excipient.

### Advantageous Effects of the Invention

The base editing system provided in the present disclosure can concurrently knock out the *TRAC*, *TGFBR2*, *PD-1*, *CISH*, and *FAS* genes (namely, editing the genes associated with enhancement of T-cells) to meet the requirements for optimizing the engineered T-cells (*e.g.*, CAR-T-cells).

### BRIEF DESCRIPTION OF THE DRAWINGS

FIG. 1: Schematic diagram of the editing efficiency results of preferred target sites in the *CISH* gene.
FIG. 2: Schematic diagram of the editing efficiency results of preferred target sites in the *FAS* gene.
FIG. 3: Schematic diagram of the editing efficiency results of preferred target sites in the *PD-1* gene.
FIG. 4: Schematic diagram of the editing efficiency results of preferred target sites in the *TGFBR2* gene.
FIG. 5: Schematic diagram of the editing efficiency results of preferred target sites in the *TRAC* gene.
FIG. 6: Schematic diagram of the editing efficiency results of mini-Sdd9-QBEmax and mini-Sdd9-BE4max on the five genes.
FIG. 7: Schematic diagram of the indel (insertion/deletion) efficiency results of mini-Sdd9-QBEmax and mini-Sdd9-BE4max on the five genes.
FIG. 8: Schematic diagram of the editing efficiency/indel results of mini-Sdd9-QBEmax and mini-Sdd9-BE4max on the five genes.
FIG. 9: Schematic diagram of the editing efficiency results of Sdd7-QBEmax and Sdd7-BE4max on the five genes.
FIG. 10: Schematic diagram of the indel efficiency results of Sdd7-QBEmax and Sdd7-BE4max on the five genes.
FIG. 11: Schematic diagram of the editing efficiency/indel results of Sdd7-QBEmax and Sdd7-BE4max on the five genes.
FIG. 12: Schematic diagram of the editing efficiency results of Sdd7-BE4max for simultaneous knockout of the five genes.
FIG. 13: Schematic diagram of the editing efficiency results of Sdd7-QBEmax for simultaneous knockout of the five genes.
FIG. 14: Schematic diagram of the editing efficiency results of mini-Sdd9-BE4max for simultaneous knockout of the five genes.
FIG. 15: Schematic diagram of the editing efficiency results of mini-Sdd9-QBEmax for simultaneous knockout of the five genes.
FIG. 16: Schematic diagram of the editing efficiency/editing purity results of mini-Sdd9-QBEmax and mini-Sdd9-BE4max for the *CISH* gene knockout, where the arrows indicate the editing sites for knockout.
FIG. 17: Schematic diagram of the editing efficiency/editing purity results of mini-Sdd9-QBEmax and mini-Sdd9-BE4max for the *FAS* gene knockout, where the arrows indicate the editing sites for knockout.
FIG. 18: Schematic diagram of the editing efficiency/editing purity results of mini-Sdd9-QBEmax and mini-Sdd9-BE4max for the *PD-1* gene knockout, where the arrows indicate the editing sites for knockout.
FIG. 19: Schematic diagram of the editing efficiency/editing purity results of mini-Sdd9-QBEmax and mini-Sdd9-BE4max for the *TGFBR2* gene knockout, where the arrows indicate the editing sites for knockout.
FIG. 20: Schematic diagram of the editing efficiency/editing purity results of mini-Sdd9-QBEmax and mini-Sdd9-BE4max for the *TRAC* gene knockout, where the arrows indicate the editing sites for knockout.
FIG. 21: Schematic diagram of the relative positions of protospacers/target sites in the five genes.
FIG. 22: Schematic diagram of the polypeptide structures of the BE4max and QBEmax base editing systems, where the promoter, NLS, and UGI are selected based on the well-known technologies in the art.

### DETAILED DESCRIPTION

### I. Definitions

In the present disclosure, the scientific and technical terms used herein have the meaning as commonly understood by those skilled in the art unless otherwise specified. Also, the terms related to the protein and nucleic acid chemistry, molecular biology, cell and tissue culture, microbiology, and immunology as well as the laboratory procedures described herein are terms and conventional steps widely used in the pertinent fields. For example, standard recombinant DNA and molecular cloning techniques employed in the present disclosure are well known to those skilled in the art and are described in greater details in the following literature: Sambrook, J., Fritsch, E.F. and Maniatis, T., Molecular Cloning: A Laboratory Manual; Cold Spring Harbor Laboratory Press: Cold Spring Harbor, 1989 (hereinafter referred to as "Sambrook"). Meanwhile, to facilitate understanding of the present disclosure, definitions and explanations of the relevant terms are described below.

As used herein, the term "and/or" encompasses all combinations of the items connected by this term, and should be regarded as if each of the combinations has been separately listed herein. For example, "A and/or B" encompasses "A", "A and B", and "B". For example, "A, B and/or C" encompasses "A", "B", "C", "A and B", "A and C", "B and C", and "A and B and C".

The term "comprising", when used herein to describe the sequence of a protein or nucleic acid, means that the protein or nucleic acid may consist of the sequence, or may have an additional amino acid or nucleotide at either or both ends, while still retaining the activity described in the present disclosure. Furthermore, those skilled in the art are aware that methionine encoded by a start codon at the N-terminus of a polypeptide will be retained under certain practical circumstances (*e.g.*, when expressed in a particular expression system), without any substantive impact on the functions of the polypeptide. Therefore, when describing the amino acid sequence of a specific polypeptide in the specification and claims of the present application, even if the sequence may not comprise the methionine encoded by a start codon at the N-terminus, the sequence containing this methionine is also encompassed herein at this time, and correspondingly, its encoding nucleotide sequence may also comprise a start codon; and vice versa.

"Gene" or "genome", as used herein, encompasses not only chromosomal DNA present in the nucleus, but also organellar DNA present in the subcellular components (e.g. mitochondria and plastids) of the cell.

"Genetically modified organism" or "genetically modified cell" means an organism or cell containing, within its genome, an exogenous polynucleotide or a modified gene or expression regulatory sequence. For example, the exogenous polynucleotide may be stably integrated into the genome of the organism or cell and inherited through successive generations. The exogenous polynucleotide may be integrated into the genome individually or as part of a recombinant DNA construct. The modified gene or expression regulatory sequence means that the sequence comprises one or more deoxynucleotide substitutions, deletions and additions in the organism or cell genome.

"Polynucleotide", "nucleic acid sequence", "nucleotide sequence", and "nucleic acid fragment" may be used interchangeably to denote a single-stranded or double-stranded RNA or DNA polymer, which may optionally contain synthetic, non-natural or altered nucleotide bases. Nucleotides are designated by their single-letter names as follows: "A" denotes adenosine or deoxyadenosine (corresponding to RNA or DNA, respectively), "C" denotes cytidine or deoxycytidine, "G" denotes guanosine or deoxyguanosine, "U" denotes uridine, "T" denotes deoxythymidine, "R" denotes purine (A or G), "Y" denotes pyrimidine (C or T), "K" denotes G or T, "H" denotes A or C or T, "I" denotes inosine, and "N" denotes any nucleotide.

"Polypeptide", "peptide", and "protein" are used interchangeably in the present disclosure to denote a polymer of amino acid residues. These terms apply to amino acid polymers in which one or more amino acid residues are artificial chemical analogue(s) of the corresponding naturally occurring amino acid(s) and to naturally occurring amino acid polymers. The terms "polypeptide", "peptide," "amino acid sequence", and "protein" may also include modified forms thereof, including but not limited to: glycosylation, lipid ligation, sulfation, γ-carboxylation of glutamic acid residues, hydroxylation, and ADP-ribosylation.

Sequence "identity" has a meaning generally recognized in the art, and the percent sequence identity between two nucleic acid or polypeptide molecules or regions can be calculated using publicly available techniques. The sequence identity may be measured along the full length of a polynucleotide or polypeptide or along a region of the molecule. (See, *e.g.*: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; Sequence Analysis Primer, Gribskov, M. and Devereux, J., eds., M Stockton Press, New York, 1991.) Although there are many methods for measuring the identity between two polynucleotides or polypeptides, the term "identity" is well known to those skilled in the art (Carrillo, H. & Lipman, D., SIAM J Applied Math 48:1073 (1988)).

In peptides or proteins, suitable conservative amino acid substitutions are known to those skilled in the art and can generally be made without altering the biological activity of the resulting molecule. Typically, those skilled in the art will recognize that substituting a single amino acid in a non-essential region of a polypeptide does not substantially alter the biological activity (see, *e.g.*, Watson et al., Molecular Biology of the Gene, 4th Edition, 1987, The Benjamin/Cummings Pub. co., p.224).

"RNA-dependent DNA polymerase" refers to an enzyme capable of synthesizing a DNA strand using RNA as a template. It can recognize the base sequence on an RNA strand and synthesize a complementary DNA sequence on the DNA strand.

"Domain" refers to a portion of a protein that has a relatively stable three-dimensional structure. A protein is typically composed of one or more domains, each having its own spatial folded conformation. Domains may be contiguous or non-contiguous and are typically composed of one or more segments of an amino acid sequence. The presence of domains renders proteins functionally diverse and enables new functions through recombination and combination of domains.

"Expression construct" or "construct" refers to a vector (such as a recombinant vector) suitable for expression of a nucleotide sequence of interest in an organism. The "expression" refers to production of a functional product. For example, the expression of a nucleotide sequence may refer to the transcription of a nucleotide sequence (*e.g.* transcription to produce mRNA or functional RNA) and/or the translation of RNA into a precursor or a mature protein. The "expression construct" may be a linear nucleic acid fragment, a circular plasmid, a viral vector or, in some embodiments, a translatable RNA (such as mRNA). The "expression construct" may include regulatory sequences and nucleotide sequences of interest that are derived from different origins, or regulatory sequences and nucleotide sequences of interest that, while derived from the same origin, are arranged in a different manner from the conventional native one.

"Introducing" a nucleic acid molecule (*e.g.*, a plasmid, a linear nucleic acid fragment, an RNA, etc.) or a protein into an organism means transforming cells of an organism with the nucleic acid or protein, such that the nucleic acid or protein can function in the cells. The "transformation" as used in the present disclosure includes stable transformation and transient transformation.

"Stable transformation" means introducing an exogenous nucleotide sequence into a genome to cause stable inheritance of the exogenous nucleotide sequence. Once stably transformed, the exogenous nucleic acid sequence is stably integrated into the genomes of the organism and any successive generations thereof.

"Transient transformation" means introducing a nucleic acid molecule or protein into a cell, such that it functions but without stable inheritance of the exogenous nucleotide sequence. In transient transformation, the exogenous nucleic acid sequence is not integrated into the genome.

"T-cell" refers to a type of lymphocyte that develops in the thymus and plays a central role in immune responses. With the presence of a T-cell receptor on the cell surface, the T-cells can be distinguished from other lymphocytes.

"Engineered T-cell" refers to any T-cell containing a cell receptor heterologous to the T-cell. For example, a T-cell containing a chimeric antigen receptor (CAR); or a T-cell containing a heterologous TCR that binds to an antigen, such as MART1, NY-ESO-1, p53, and analogs thereof.

"Chimeric antigen receptor", "CAR" or "CARs" is a term understood by those skilled in the art and refers to a class of antigen-binding proteins that are fusion proteins comprising a selected single-chain fragment of a variable domain derived from a specific monoclonal antibody and one or more intracellular signaling domains of a T-cell receptor.

"CAR-T-cell therapy" refers to a type of treatment that involves genetic modification of a patient's autologous T-cells to make them express a CAR specific to a tumor antigen, followed by *ex vivo* cell proliferation and reinfusion back into the patient. Such a T-cell genetic modification may be realized by virus-based gene transfer methods or non-viral methods, such as DNA-based transposons, the CRISPR/Cas9 technology, or direct transfer of *in vitro* transcribed mRNA by electroporation.

"GvHD" refers to a syndrome characterized by inflammation in various organs, with the specificity to epithelial cell apoptosis and crypt dropout. GvHD is often associated with stem cell transplantation (such as those occurring in bone marrow transplantation), and may also be induced by CAR therapies based on donor leukocyte infusion, virus-specific T-cells, T-cell receptor-deficient T-cells, lymphoid progenitor cells, and regulatory T-cells.

"HEK293T" refers to a variant of the human embryonic kidney 293 (HEK293) cell containing the SV40 large T-antigen. This antigen allows the episome of a transfected plasmid containing the SV40 origin of replication to replicate, which leads to amplification of the transfected plasmid and prolonged expression of the desired gene product.

"*In vitro*" refers to a method performed outside a living organism, typically using a biological material isolated from its normal biological host or natural biological environment.

"Ortholog" or "orthologous sequence" refers to a homologous nucleic acid or amino acid sequence derived from the same ancestral sequence separated by a speciation event.

"Homolog" or "homologous sequence" refers to a nucleic acid or amino acid sequence derived from a common ancestral sequence.

### II. Base Editing System of Modifying T-cells

In one aspect, the present disclosure provides a base editing system of modifying a T-cell, comprising:
i) a base editing polypeptide, and/or an expression construct encoding the base editing polypeptide, wherein the base editing polypeptide comprises a DNA-binding protein and at least one deaminase domain; and
ii) a guide RNA, and/or an expression construct encoding the guide RNA, wherein the guide RNA targets any one or any combination of a T-cell receptor alpha chain constant (TRAC) gene, a transforming growth factor beta receptor 2 (TGFBR2) gene, a programmed death receptor 1 (PD-1) gene, a cytokine-inducible SH2-containing protein (CISH) gene, and/or a cell suicide-associated factor (FAS) genomic region.

"DNA-binding protein" refers to a protein associated with a nucleic acid (*e.g.*, DNA or RNA). For example, a DNA-binding protein may be guided to a specific nucleic acid sequence via a guide RNA. In some embodiments, the DNA-binding protein is a Transcription Activator-Like Effector (TALE), a Zinc Finger Protein (ZFP) or a CRISPR effector protein. In some embodiments, the CRISPR effector protein is Cas3, Cas4, Cas5, Cas5e (or CasD), Cas6, Cas6e, Cas6f, Cas7, Cas8a1, Cas8a2, Cas8b, Cas8c, Cas9, Cas10, Cast10d, Cas12, Cas13, Cas14, CasX, CasF, CasG, CasH, Csy1, Csy2, Csy3, Cse1 (or CasA), Cse2 (or CasB), Cse3 (or CasE), Cse4 (or CasC), Csc1, Csc2, Csa5, Csn1, Csn2, Csm2, Csm3, Csm4, Csm5, Csm6, Cmrl, Cmr3, Cmr4, Cmr5, Cmr6, Cpf1, Csb1, Csb2, Csb3, Csx17, Csx14, Csx10, Csx16, CsaX, Csx3, Csz1, Csx15, Csf1, Csf2, Csf3, Csf4, Cu1966, TraC, or a variant thereof.

In some embodiments, the DNA-binding protein is a nickase or a variant thereof. "Nicking enzyme" or "Nickase" is a special enzyme, which is an endonuclease, capable of recognizing a specific sequence on the double-stranded DNA and creating a nick on only one of the two strands, without completely cleaving the double-stranded DNA. The nick is typically created at a phosphodiester bond, resulting in an open end of the DNA while leaving the other strand intact. For example, in the CRISPR/Cas9 gene editing technology, a nickase version of Cas9 (referred to as nCas9 or Cas9n) is used to create single-strand nicks rather than double-strand breaks. For example, residues (such as Asp10, His840, Asn854, and Asn856) in an exemplary wild-type *Streptococcus pyogenes* Cas9 polypeptide may be mutated to deactivate one of the multiple nucleic acid cleavage domains (*e.g.*, nuclease domains). In some embodiments, the DNA-binding protein is a Cas9 nickase or a circularly permuted polypeptide other than deaminases used in QBEmax.

In some embodiments, the DNA-binding protein is a deactivated and/or conditionally deactivated nuclease or a variant thereof. A deactivated nuclease can target a nucleic acid without causing strand breaks. For example, residues (such as Asp10, His840, Asn854, and Asn856) in an exemplary wild-type *Streptococcus pyogenes* Cas9 polypeptide may be mutated to deactivate the nucleic acid cleavage domains of the multiple nucleic acid cleavage domains (*e.g.*, nuclease domains). In some embodiments, the DNA-binding protein is a deactivated Cas9 (dCas9).

As used herein, "deaminase domain" refers to an enzyme that catalyzes a deamination reaction. In some embodiments, the deaminase is a cytidine deaminase or an adenosine deaminase, *i.e.*, an enzyme capable of removing an amino group from a cytidine or adenosine molecule. In some embodiments, the deaminase is an APOBEC family deaminase, an SCP1.201 family deaminase or a homolog thereof; the APOBEC family deaminase is selected from an AID deaminase, an APOBEC1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, and an APOBEC3H deaminase; the SCP1.201 family deaminase is an Sdd2 deaminase, an Sdd3 deaminase, an Sdd4 deaminase, an Sdd6 deaminase, an Sdd7 deaminase, a mini-Sdd7 deaminase, a mini-Sdd9 deaminase, an Sdd10 deaminase, an Sdd59 deaminase, a mini-Sdd3 deaminase, a mini-Sdd6 deaminase, or their respective functional mutants. Non-limiting examples of the SCP1.201 family deaminase include the amino acid sequences SEQ ID NOs: 70-73 and SEQ ID NOs: 81-88.

In some embodiments, the base editing system of the present disclosure further contains a uracil DNA glycosylase inhibitor (UGI). Its non-limiting examples include the amino acid sequence SEQ ID NO: 89.

In some embodiments, the base editing system of the present disclosure further comprises a nuclear localization sequence. As used herein, "nuclear localization sequence (NLS)" or "nuclear localization signal" may be used interchangeably to denote a domain of a protein (typically a short amino acid sequence) capable of interacting with a nuclear vector to have the protein transported into the cell nucleus. Generally, one or more NLSs in the base editing system (*e.g.*, a base editing polypeptide) should have sufficient strength to drive the base editing system (*e.g.*, a base editing polypeptide) to accumulate in the nucleus of a cell in an amount sufficient to achieve its base editing function. Generally, the strength of nuclear localization activity is determined by the number and location of NLSs of the base editing system (*e.g.*, a base editing polypeptide), one or more specific NLSs used, or a combination of these factors. In some embodiments of the present disclosure, the NLS of the base editing system (*e.g.*, a base editing polypeptide) of the present disclosure may be located at the N-terminus and/or C-terminus. In some embodiments of the present disclosure, the NLS of the base editing system (*e.g.*, a base editing polypeptide) of the present disclosure may be located between the deaminase domain (*e.g.*, adenosine deaminase domain and/or cytidine deaminase domain), the DNA-binding protein and/or UGI. In some embodiments, the base editing system (*e.g.*, a base editing polypeptide) includes about 1, 2, 3, 4, 5, 6, 7, 8, 9, 10 or more NLSs. In some embodiments, the base editing system (*e.g.*, a base editing polypeptide) includes about 1, 2, 3, 4, 5, 6, 7, 8, 9, 10 or more NLSs at or near the N-terminus. In some embodiments, the base editing system (*e.g.*, a base editing polypeptide) includes about 1, 2, 3, 4, 5, 6, 7, 8, 9, 10 or more NLSs at or near the C-terminus. In some embodiments, the polypeptide comprises a combination of these, such as one or more NLSs at the N-terminus and one or more NLSs at the C-terminus. When more than one NLS is present, each may be selected to be independent of the other NLSs. Generally, an NLS is composed of one or more short sequences of positively charged lysine or arginine exposed on the protein surface, but other types of NLSs are also known. Non-limiting examples of NLS include the amino acid sequences of SEQ ID NOs: 52-68.

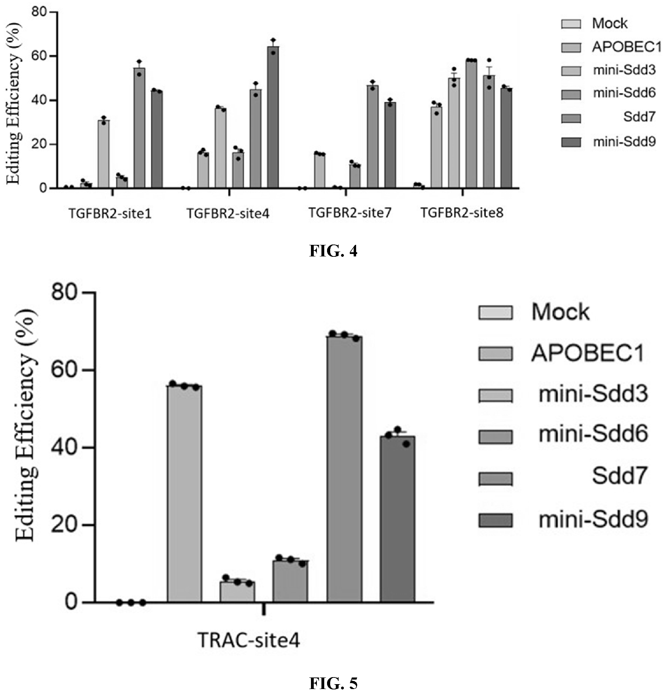

"Base editing polypeptide" refers to a polypeptide that binds a polynucleotide and has base-modifying activity. In some embodiments, the base editing polypeptide comprises a DNA-binding protein and at least one deaminase domain. In some embodiments, the framework of the base editing polypeptide is a BE system, such as BE1, BE2, BE3 or BE4max (reference: Komor A C, Zhao K T, Packer M S, et al. Improved base excision repair inhibition and bacteriophage Mu Gam protein yields C: G-to-T: A base editors with higher efficiency and product purity[J]. Science advances, 2017, 3(8): eaao4774.; BE4max is BE4 in this reference, whose structure is shown in Figure 5A of this reference) or QBEmax.

In the present specification, the Base Editor (BE) system includes BE1, BE2, BE3, BE4, etc. Among them, BE1 is composed of a fusion protein of dCas9 and APOBEC1. BE2 is a BE system obtained by introducing UGI (Uracil DNA glycosylase inhibitor) into BE1. BE3 refers to a BE system obtained by replacing dCas9 in BE2 with nCas9 (D10A). BE4 refers to a BE system obtained from BE3 by optimizing the length of the linker region between APOBEC1 and nCas9 (D10A), optimizing the length of the linker region between nCas9 (D10A) and UGI, and adding one copy of UGI.

In some embodiments, QBEmax includes a deaminase domain and paired DNA-binding protein domains having a single-strand DNA cleavage function.

In some embodiments of the present disclosure, the DNA-binding protein having a single-strand DNA cleavage activity consists of two paired DNA-binding protein domains that are respectively linked via linkers to the N-terminus and the C-terminus of the deaminase domain. In some embodiments of the present disclosure, the paired DNA-binding protein domains can be obtained by subjecting the amino acid sequence of a DNA-binding protein having a single-strand cleavage function to circular permutation (the linker for circular permutation may be GGSGGSGGSGGSGGSGGSGG; SEQ ID NO: 90) to produce a circularly permuted sequence of 1388 amino acids (the circularly permuted sequence is as set forth in SEQ ID NO: 91, where the amino acid position of the circularly permuted sequence is defined in such a way that the methionine M encoded by a start codon is the first amino acid, and the glycine G directly linked to the first amino acid in the linker is the 1388^{th} amino acid). The circularly permuted DNA-binding protein is cleaved at two sites to obtain a pair of separate polypeptides, which are the paired DNA-binding protein domains having a single-strand DNA cleavage function. The above paired DNA-binding protein domains having a single-strand DNA cleavage function are respectively fused or linked to the N-terminus and the C-terminus of the deaminase domain. Depending on their positions at which they are fused to the deaminase, the domains linked to the N-terminus or the C-terminus of the deaminase are defined as the deaminase N-terminal linker domain (QBE-N) and the deaminase C-terminal linker domain (QBE-C).

Exemplarily:

| Deaminase terminal linker domain | First cleavage site | Second cleavage site | Sequence of deaminase N-terminal linker domain (QBE-N) | Sequence of deaminase C-terminal linker domain (QBE-C) |
|---|---|---|---|---|
| QBEmax (also called QBE3) | 1244/1245 | 1030/1031 | SEQ ID NO: 92 | SEQ ID NO: 93 |

The first cleavage site is defined as a deaminase linking site, where the lower-numbered amino acid is linked to the N-terminus of the deaminase and the higher-numbered amino acid is linked to the C-terminus of the deaminase; and the second cleavage site is defined as a non-deaminase linking site, where the lower-numbered amino acid is the C-terminus of the QBE-C domain and the higher-numbered amino acid is the N-terminus of the QBE-N domain.

Therefore, in some embodiments, the paired DNA-binding protein domains having a single-strand DNA cleavage function include a deaminase N-terminal linker domain and a deaminase C-terminal linker domain that are respectively linked via linkers to the N-terminus and C-terminus of a deaminase domain.

In some embodiments, the N-terminus or C-terminus of the deaminase domain includes one or two single domains that are linked via a linker. As used herein, "single domain" is a portion of the DNA-binding protein domain, and all single domains are assembled into a complete DNA-binding protein domain. The single domain does not contain a linker, but has linkers at both ends that link it to other single domains or functional regions (such as deaminase, NLS or UGI).

In some embodiments, QBEmax comprises the following structure: [X₂]-[ deaminase]- [X₃]- [X₄], where "-" represents a linker or a circularly permuted linker, and the linker has a length of 0 to 100 amino acids.

In some embodiments, X₂₋₄ are single domains. In some preferred embodiments, X₂ is SEQ ID NO: 94, X₃ is SEQ ID NO: 95, and X₄ is SEQ ID NO: 96.

The deaminase is as described above for "deaminase domain".

As used herein, "linker" refers a linker that links two molecules or moieties, such as two domains of a fusion protein. Typically, a linker is located between or flanks two groups, molecules or other moieties, and is linked thereto via a covalent bond, thereby connecting the two. In some embodiments, the linker is an organic molecule, group, polymer, or chemical moiety.

In some embodiments, the base editing polypeptide comprises an amino acid sequence having at least 80% sequence identity to any one of SEQ ID NOs: 74-80. In some embodiments, the base editing polypeptide comprises an amino acid sequence having at least 80% sequence identity to any one of SEQ ID NOs: 77-80.

"Guide RNA" and "gRNA" may be used interchangeably to denote a polynucleotide that is specific to a target sequence of a target nucleic acid and is capable of forming a complex with a DNA-binding protein.

In the present disclosure, "target nucleic acid" is a polynucleotide (*e.g.*, DNA such as genomic DNA) comprising a site ("target site" or "target sequence") targeted by a DNA-binding protein (*e.g.*, a Cas protein) guided by the guide RNA. The "target sequence" is a sequence with which the guide sequence of the guide RNA will hybridize.

In the present specification, "protospacer", and "editing window" may be used interchangeably to denote a nucleic acid sequence within a genome that is complementary to the target sequence. In the native state, the protospacer sequence is adjacent to a protospacer adjacent motif (PAM). During the base editing, nucleotides within this sequence are subjected to the deamination reaction and the nucleotide substitution under the action of the base editing system.

In the present specification, "spacer" is an RNA sequence within the guide sequence of gRNA, which is complementary to and hybridizable with a target sequence, *i.e.*, the RNA version of the protospacer sequence.

In some embodiments, the guide RNA is present as a single RNA molecule. In some embodiments, the guide RNA is an sgRNA comprising two portions: (1) a guide sequence complementary to the target sequence in a target nucleic acid; and (2) a portion that binds a DNA-binding protein.

In some embodiments, the guide sequence of portion (1) comprises a spacer sequence corresponding to the protospacers in the TRAC, TGFBR2, PD-1, CISH, and FAS genomes, and the spacer sequence is complementary to the target sequence.

As used herein, "site1, site2 ..." represent protospacer sequences. In some specific embodiments, the protospacer sequences are as set forth in SEQ ID NOs: 1-51.

In some specific embodiments, the target sequence is a DNA sequence complementary to the protospacer sequence as set forth in SEQ ID NOs: 1-51 (it can be appreciated that the target sequence is located on the non-editing strand). As used herein, "sg1, sg2 ..." represent RNA sequences (guide sequences) complementary to the target sequence, *i.e.*, the RNA sequences complementary to the DNA sequences that are complementary to SEQ ID NOs: 1-51.

In some embodiments, the portion (2) corresponds to a sequence referred to as the scaffold sequence, which is responsible for specifically binding to the DNA-binding protein. In some embodiments, the portion (2) is identical or homologous to the scaffold sequence provided in Jinek et al., Science 337:816-821(2012) or Nguyen et al., Science 386, eado9336 (2024). In some embodiments, the scaffold sequence is as set forth in SEQ ID NOs: 97-99.

In some embodiments, the guide RNA comprises a spacer sequence corresponding to the protospacer sequence at the target site selected from the TRAC, TGFBR2, PD-1, CISH, and FAS genomes. The genomic sequence is referenced to GRCh38.p14.

In some optional embodiments, the protospacer sequence comprises:
(a) at least one of the nucleotide sequences as set forth in SEQ ID NOs: 1-10; and/or
(b) at least one of the nucleotide sequences as set forth in SEQ ID NOs: 11-20; and/or
(c) at least one of the nucleotide sequences as set forth in SEQ ID NOs: 21-30; and/or
(d) at least one of the nucleotide sequences as set forth in SEQ ID NOs: 31-47; and/or
(e) at least one of the nucleotide sequences as set forth in SEQ ID NOs: 48-51.

In some specific embodiments, the guide RNA contains a spacer corresponding to the following protospacer in the *TRAC*, *TGFBR2*, *PD-1*, *CISH*, or *FAS* gene:
the protospacer sequence of the *TRAC* gene comprises SEQ ID NO: 51; the protospacer sequence of the *TGFBR2* gene comprises SEQ ID NO: 21, SEQ ID NO: 24, SEQ ID NO: 27, or SEQ ID NO: 28; the protospacer sequence of the *PD-1* gene comprises SEQ ID NO: 41, SEQ ID NO: 44, SEQ ID NO: 45, SEQ ID NO: 46, or SEQ ID NO: 47; the protospacer sequence of the *CISH* gene comprises SEQ ID NO: 1, SEQ ID NO: 5, SEQ ID NO: 8, or SEQ ID NO: 10; and the protospacer sequence of the *FAS* gene comprises SEQ ID NO: 14, SEQ ID NO: 17, SEQ ID NO: 18, or SEQ ID NO: 20.

In some preferred embodiments, the protospacer sequence of the *TRAC* gene comprises SEQ ID NO: 51; the protospacer sequence of the *TGFBR2* gene comprises SEQ ID NO: 24 or SEQ ID NO: 28; the protospacer sequence of the *PD-1* gene comprises SEQ ID NO: 41 or SEQ ID NO: 47; the protospacer sequence of the *CISH* gene comprises SEQ ID NO: 1; and the protospacer sequence of the *FAS* gene comprises SEQ ID NO: 14.

### III. Method for Obtaining Modified T-cells

In another aspect, the present disclosure provides a method for obtaining a modified T-cell, wherein the method comprises knocking out or downregulating expression of an endogenous gene in a T-cell by a gene editing technology, wherein the endogenous gene comprises a T-cell receptor alpha chain constant (TRAC) gene, a transforming growth factor beta receptor 2 (TGFBR2) gene, a programmed death receptor 1 (PD-1) gene, a cytokine-inducible SH2-containing protein (CISH) gene, and a cell suicide-associated factor (FAS) gene.

In some embodiments, the gene editing technology comprises DNA insertion, deletion or substitution at a gene target, and preferably, the gene editing technology is DNA substitution at a gene target.

In some specific embodiments, the gene editing technology comprises single-base editing.

In some specific embodiments, the method comprises:
a) providing a purified T-cell population;
b) introducing a single-base editing system into the T-cell population of a); and
c) amplifying and harvesting the modified T-cell.

In some embodiments, the single-base editing system in step b) comprises: i) a base editing polypeptide, and/or an expression construct encoding the base editing polypeptide; and ii) a guide RNA (gRNA) containing a spacer corresponding to a protospacer in the *TRAC*, *TGFBR2*, *PD-1*, *CISH* or *FAS* gene, and/or an expression construct encoding the guide RNA.

In some embodiments, the protospacer sequence of the *TRAC* gene comprises at least one of the nucleotide sequences as set forth in SEQ ID NOs: 48-51; the protospacer sequence of the *TGFBR2* gene comprises at least one of the nucleotide sequences as set forth in SEQ ID NOs: 21-30; the protospacer sequence of the *PD-1* gene comprises at least one of the nucleotide sequences as set forth in SEQ ID NOs: 31-47; the protospacer sequence of the *CISH* gene comprises at least one of the nucleotide sequences as set forth in SEQ ID NOs: 1-10; and the protospacer sequence of the *FAS* gene comprises at least one of the nucleotide sequences as set forth in SEQ ID NOs: 11-20.

In some preferred embodiments, the protospacer sequence of the *TRAC* gene comprises SEQ ID NO: 51; the protospacer sequence of the *TGFBR2* gene comprises SEQ ID NO: 21, SEQ ID NO: 24, SEQ ID NO: 27, or SEQ ID NO: 28; the protospacer sequence of the *PD-1* gene comprises SEQ ID NO: 41, SEQ ID NO: 44, SEQ ID NO: 45, SEQ ID NO: 46, or SEQ ID NO: 47; the protospacer sequence of the *CISH* gene comprises SEQ ID NO: 1, SEQ ID NO: 5, SEQ ID NO: 8, or SEQ ID NO: 10; and the protospacer sequence of the *FAS* gene comprises SEQ ID NO: 14, SEQ ID NO: 17, SEQ ID NO: 18, or SEQ ID NO: 20.

In some more preferred embodiments, the protospacer sequence of the *TRAC* gene comprises SEQ ID NO: 51; the protospacer sequence of the *TGFBR2* gene comprises SEQ ID NO: 24 or SEQ ID NO: 28; the protospacer sequence of the *PD-1* gene comprises SEQ ID NO: 41 or SEQ ID NO: 47; the protospacer sequence of the *CISH* gene comprises SEQ ID NO: 1; and the protospacer sequence of the *FAS* gene comprises SEQ ID NO: 14.

In some embodiments, the base editing polypeptide comprises a DNA-binding protein and at least one deaminase domain.

In some embodiments, the base editing polypeptide further comprises at least one uracil DNA glycosylase inhibitor (UGI) domain.

In some embodiments, the base editing polypeptide further comprises a nuclear localization sequence.

In some embodiments, the deaminase is a cytidine deaminase; optionally, the cytidine deaminase is an SCP1.201 family deaminase; alternatively, the SCP1.201 family deaminase is an Sdd2 deaminase, an Sdd3 deaminase, an Sdd4 deaminase, an Sdd6 deaminase, an Sdd7 deaminase, a mini-Sdd7 deaminase, a mini-Sdd9 deaminase, an Sdd10 deaminase, an Sdd59 deaminase, a mini-Sdd3 deaminase, or a mini-Sdd6 deaminase.

In some embodiments, the deaminase is Sdd7 and/or mini-Sdd9 cytidine deaminase(s).

In some embodiments, the DNA-binding protein is a TALE, ZFP or CRISPR effector protein or a variant thereof; optionally, the CRISPR effector protein is a Cas9 nickase, a deactivated Cas9, or a variant thereof.

In some embodiments, the base editing polypeptide comprises an amino acid sequence having at least 80%, 85%, 90%, 95%, 96%, 97%, 98%, or 99% sequence identity to any one of SEQ ID NOs: 74-80.

In some embodiments, the method further comprises introducing a nucleic acid sequence encoding a chimeric antigen receptor (CAR), wherein the chimeric antigen receptor (CAR) comprises an antigen-binding domain and a transmembrane domain.

In some embodiments, the T-cell population is T-cells derived from humans, optionally T-cells enriched from human blood cells, and alternatively cryopreserved T-cells enriched from human blood cells.

In some embodiments, the method for the introduction comprises electroporation, liposome transfection, microinjection, viral infection (such as adenovirus, adeno-associated virus, lentivirus, or other viruses), N-acetylgalactosamine (GalNAc)-mediated transformation, gene gun-mediated transformation, ribonucleoprotein (RNP) complex-mediated transformation, and nanoparticle-mediated transformation.

In some embodiments, the single-base editing system is a base editing system of modifying T-cells described in the above "**II. Base Editing System of Modifying T-cells**". The method comprises introducing the base editing system into T-cells *in vitro* or *ex vivo*. In some embodiments, the method comprises knocking out or introducing a dominant deactivation mutation into the *TRAC*, *TGFBR2*, *PD-1*, *CISH*, and *FAS* genes. In some embodiments, the base editing system is introduced into T-cells (*e.g.*, CAR-T-cells) by the methods selected from: electroporation, liposome transfection, microinjection, viral infection (such as adenovirus, adeno-associated virus, lentivirus, or other viruses), N-acetylgalactosamine (GalNAc)-mediated transformation, gene gun-mediated transformation, ribonucleoprotein (RNP) complex-mediated transformation, and nanoparticle-mediated transformation.

The base editing system according to the present disclosure may be transfected or injected into cells, tissues, or organs in the form of DNA, RNA, protein, virus, or the like.

In some embodiments, a gene delivery vector may be used to deliver a polynucleotide (*e.g.*, a polynucleotide encoding the base editing system described herein) to a cell or tissue. As used herein, "gene delivery", "gene transfer", "transduction", or the like refer to introduction of an exogenous polynucleotide into a host cell, for example, a vector-mediated gene transfer (by, *e.g.*, viral infection/transfection, or various other protein- or lipid-based gene delivery complexes) and techniques that assist in the delivery of a "naked" polynucleotide (*e.g.*, electroporation, "gene gun" delivery, and various other techniques for the introduction of polynucleotides). The introduced polynucleotide may be maintained stably or transiently in the host cell. Stable maintenance typically requires that the introduced polynucleotide comprises an origin of replication compatible with the host cell or is incorporated into a replicon of the host cell, such as an extrachromosomal replicon (*e.g.*, a plasmid) or a nuclear or mitochondrial chromosome. Many "vectors" are known to be able to mediate the transfer of a gene into a mammalian cell, as known in the art and described herein.

### IV. Modified T-cell

In another aspect, the present disclosure provides a modified T-cell. The T-cell has been modified by the method described in the above "**III. Method for Obtaining Modified T-cells**", or introduced with the base editing system described in the above "**II. Base Editing System of Modifying T-cells**".

In some embodiments, the endogenous gene in the T-cell is knocked out or the expression of the endogenous gene in the T-cell is downregulated, wherein the endogenous gene comprises a T-cell receptor alpha chain constant (TRAC) gene, a transforming growth factor beta receptor 2 (TGFBR2) gene, a programmed death receptor 1 (PD-1) gene, a cytokine-inducible SH2-containing protein (CISH) gene, and a cell suicide-associated factor (FAS) gene.

In some embodiments, the T-cell is a CAR-T-cell.

In some specific embodiments, the CAR-T-cell recognizes one or more target antigens.

In some specific embodiments, the CAR-T-cell comprises a chimeric antigen receptor. The preparation method or composition of the chimeric antigen receptor is existing technology and is not specifically limited in the present disclosure. For example, the chimeric antigen receptor comprises a transmembrane domain, an intracellular domain, and an extracellular domain. The extracellular domain comprises an antibody that targets an antigen, and the specific type of antibody may be selected based on the antigen. In some embodiments, the CAR-T-cell is an allogeneic CAR-T-cell or an autologous CAR-T-cell (*e.g.*, an autologous human CAR-T-cell).

The CAR-T-cells provided in the present disclosure can be used for preventing or treating an autoimmune disease, a tumor, a viral infectious disease, a bacterial infectious disease, a genetic disease, an infectious disease, or a cardiovascular disease.

The autoimmune disease is one or more selected from systemic lupus erythematosus, rheumatoid arthritis, psoriatic arthritis, lupus nephritis, neuromyelitis optica, systemic sclerosis, Sjögren's syndrome, and polymyositis.

The tumor is lymphoma, hematological tumor or solid tumor; and preferably is one or more selected from adrenocortical carcinoma, bladder urothelial carcinoma, breast cancer, cervical squamous cell carcinoma, endocervical adenocarcinoma, cholangiocarcinoma, colon adenocarcinoma, lymphoid neoplasms, diffuse large B-cell lymphoma, esophageal cancer, glioblastoma multiforme, head and neck squamous cell carcinoma, chromophobe renal cell carcinoma, clear cell renal cell carcinoma, papillary renal cell carcinoma, acute myeloid leukemia, brain low-grade glioma, hepatocellular carcinoma, lung adenocarcinoma, lung squamous cell carcinoma, mesothelioma, ovarian cancer, pancreatic cancer, pheochromocytoma and paraganglioma, prostate cancer, rectal cancer, malignant sarcoma, melanoma, gastric cancer, testicular germ cell tumor, thyroid cancer, thymoma, endometrial cancer, uterine sarcoma, uveal melanoma, multiple myeloma, acute lymphoblastic leukemia, chronic lymphocytic leukemia, chronic myeloid leukemia, T-cell lymphoma, B-cell lymphoma, lung cancer, anal cancer, intraocular melanoma, and retinoblastoma.

The virus is one or more selected from influenza virus, parainfluenza virus, measles virus, mumps virus, herpes virus, adenovirus, respiratory syncytial virus, poliovirus, coxsackievirus, or echovirus.

The bacterium is one or more selected from *Escherichia coli*, *Lactobacillus casei*, *Bacteroides fragilis*, *Acinetobacter lwoffii*, *Fusobacterium nucleatum*, *Bacteroides johnsonii*, *Bacteroides arabidopsis*, *Lactobacillus rhamnosus*, *Bacteroides massiliensis*, *Bacteroides ovatus*, *Campylobacter jejuni*, *Staphylococcus saprophyticus*, *Enterococcus faecalis*, *Bacteroides thetaiotaomicron*, *Bacteroides vulgatus*, *Bacteroides uniformis*, *Parabacteroides faecalis*, *Fusobacterium mortiferum*, and *Bifidobacterium breve*.

The genetic disease is selected from a chromosomal disease or chromosomal syndrome, a monogenic disease, and a polygenic disease. The polygenic disease is, for example, asthma, cleftlip, schizophrenia, anencephaly, hypertension, congenital heart disease, or epilepsy.

The cardiovascular disease is selected from coronary heart disease, cerebrovascular disease, peripheral arterial vascular disease, rheumatic heart disease, congenital heart disease, deep vein thrombosis, and pulmonary embolism.

The present disclosure further provides a method for preventing or treating a disease, the method comprising administering a therapeutically effective amount of the T-cell or the CAR-T-cell to a subject in need thereof.

The present disclosure further provides a composition comprising the modified T-cell according to "**IV. Modified T-cell**", and optionally a pharmaceutically acceptable carrier or excipient.

### Examples

For the experimental methods in the following examples, reference is made to the following literatures: Shan Q, et al. Genome editing in rice and wheat using the CRISPR/Cas system. Nat Protoc. 2014; 9(10):2395-2410. doi:10.1038/nprot.2014.157; Liang Z, et al. Genome editing of bread wheat using biolistic delivery of CRISPR/Cas9 in vitro transcripts or ribonucleoproteins. Nat Protoc. 2018; 13(3):413-430. doi:10.1038/nprot.2017.145; Jin S, et al. An unbiased method for evaluating the genome-wide specificity of base editors in rice. Nat Protoc. 2021; 16(1):431-457. doi:10.1038/s41596-020-00423-y; Huang J, et al. Discovery of deaminase functions by structure-based protein clustering [published correction appears in Cell. 2024 Aug 8;187(16):4426-4428. doi: 10.1016/j.cell.2024.07.003]. Cell. 2023; 186(15):3182-3195.e14. doi:10.1016/j.cell.2023.05.041, which are incorporated herein by reference.

### Example 1. Identification of sgRNA

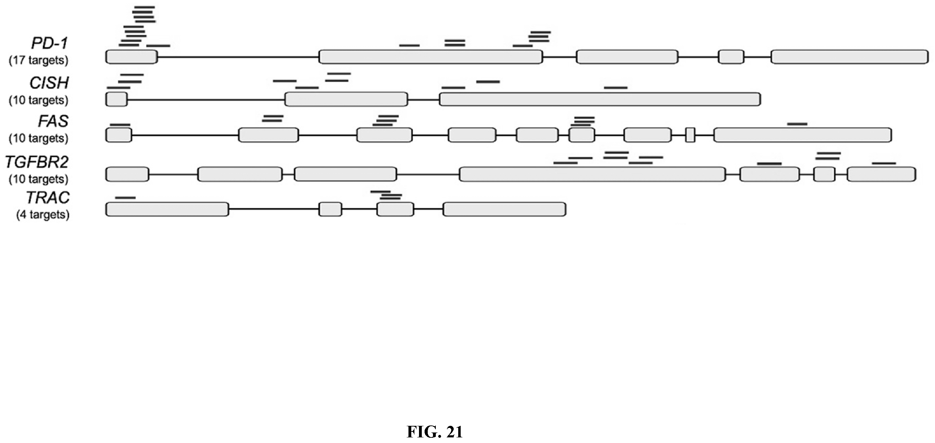

In the preparation of engineered T-cells (particularly CAR-T-cells), knocking out five genes-*PD-1*, *CISH*, *FAS*, *TGFBR2*, and *TRAC*-contributed to the survival, proliferation, persistence, activation, and tumor-killing ability of the T-cells, while effectively inhibiting or even eliminating the immune rejection or GvHD reaction. In addition to PAM, protospacer, and off-target site, the gene knockout was also constrained by the codon. The gene knockout could only be achieved by converting, using a cytidine base editor (CBE), the DNA containing a codon encoding arginine (CGA) or glutamine (CAA, CAG) or tryptophan (UGG) within the editing window into DNA containing a stop codon (UAG, UGA, UAA), or by editing an essential splice site. To this end, after comprehensive consideration of the above factors, a set of candidate editing target sites was ultimately selected from these five genes. Ten target sites were selected from each of *CISH*, *FAS*, and *TGFBR2*, and four target sites were selected from *TRAC*; seventeen target sites were selected from *PD-1*, resulting in a total of 51 target sites. The protospacer sequences for the above target sites in the target nucleic acid were as set forth in SEQ ID NOs: 1-51. The relative positions of these target sites in the genes were shown in FIG. 21. sgRNAs were designed for the above target sites, where the guide sequence of each sgRNA was complementary to the target sequence on the non-editing strand (*i.e.*, the complementary sequence of the protospacer sequence), such that the sgRNA was bound to the target sequence (the complementary sequence of the protospacer) on the non-editing strand. The scaffold sequence of the sgRNA was as set forth in SEQ ID NO: 97. Based on the consistency of the above gene sequences in the cell genome, HEK293T-cells were selected as the validation cells in this example.

Each of the above-mentioned sgRNAs complementary to the target sequences and each of the editing system plasmids containing a BE4max scaffold fused respectively to APOBEC1, mini-Sdd3, mini-Sdd6, Sdd7, and mini-Sdd9 deaminases (SEQ ID NOs: 74-78) were transfected into HEK293T-cells. The transfection conditions were as follows: cells were seeded in a 48-well plate at 60,000 cells per well, and then transfected 18-24 hours after plating. The cells were transfected as plasmids. For each well, 375 ng of the editing system plasmid and 125 ng of the sgRNA plasmid were added, mixed, and then transfected with 1 µL of jetPRIME transfection reagent (Cat. No. 101000046, Polyplus Inc.). The genomic DNA was extracted from the cells 72 hours after transfection. The target fragment was amplified by two rounds of PCR and the products were purified. The base editing efficiency at the target site was detected by next-generation sequencing (NGS).

The results were as shown in Table 1 (where "\" indicated that no editing efficiency was detected). By validating the bioinformatically screened sgRNAs in actual HEK293T-cells, the inventors found that the editing efficiency varied significantly among different protospacers, with the highest reaching 71.26% and the lowest being undetectable. Furthermore, base editing polypeptides each composed of a different deaminase exhibited significant preferences for different target sites during editing.

**Table 1**

| Name of Protospacer Sequence | Direction | Editing Site in Window | Editing Efficiency (%) | | | | |
|---|---|---|---|---|---|---|---|
| | | | APOBEC1 | mini-Sdd3 | mini-Sdd6 | Sdd7 | mini-Sdd9 |
| CISH-site1 | Sense strand | 9 | 71.26 | 3.50 | 30.03 | 65.73 | 60.42 |
| CISH-site2 | Sense strand | 9 | 1.33 | 22.06 | 7.92 | 30.76 | 41.15 |
| CISH-site3 | Sense strand | 8 | 2.53 | 14.06 | 8.88 | 44.16 | 44.58 |
| CISH-site4 | Antisense strand | 17, 18 | \ | 0.07 | \ | 0.40 | 0.31 |
| CISH-site5 | Sense strand | 7 | 5.01 | 8.34 | 9.07 | 47.46 | 41.46 |
| CISH-site6 | Antisense strand | 8, 9 | \ | 2.17 | \ | 32.80 | 14.48 |
| CISH-site7 | Sense strand | 6 | 3.26 | 10.31 | 4.08 | 40.28 | 28.71 |
| CISH-site8 | Sense strand | 7 | 10.44 | 25.68 | 50.17 | 39.57 | 30.81 |
| CISH-site9 | Sense strand | 11 | 0.38 | 2.60 | 1.42 | 6.37 | 17.22 |
| CISH-site10 | Splice donor | 5 | 49.38 | 18.97 | 36.72 | 48.44 | 42.49 |
| FAS-site1 | Sense strand | 16 | \ | \ | \ | 0.71 | 0.21 |
| FAS-site2 | Sense strand | 15 | \ | \ | \ | 2 | 1.60 |
| FAS-site3 | Sense strand | 11 | \ | 2.22 | \ | 2.56 | 22.03 |
| FAS-site4 | Antisense strand | 7, 8 | 27.27 | 8.25 | 9.37 | 31.03 | 56.74 |
| FAS-site5 | Sense strand | 14 | 1.92 | 0.10 | 1.33 | 8.85 | 1.50 |
| FAS-site6 | Sense strand | 10 | \ | 0.26 | \ | 0.27 | 0.71 |
| FAS-site7 | Antisense strand | 4, 5 | 6.74 | 26.35 | 38.90 | 61.68 | 39.73 |
| FAS-site8 | Antisense strand | 3, 4 | 32.03 | 14.11 | 40.79 | 49.05 | 40.63 |
| FAS-site9 | Antisense strand | 9, 10 | 31.32 | 5.39 | 15.60 | 28.34 | 23.37 |
| FAS-site10 | Sense strand | 6 | 24.05 | 0.70 | 3.29 | 53.45 | 14.92 |
| TGFBR2-site 1 | Sense strand | 8 | 2.32 | 31.00 | 5.17 | 54.71 | 44.38 |
| TGFBR2-site 2 | Sense strand | 11 | \ | \ | \ | 5.39 | 20.49 |
| TGFBR2-site 3 | Sense strand | 15 | \ | \ | \ | 1.26 | 2.11 |
| TGFBR2-site 4 | Sense strand | 6 | 16.38 | 36.38 | 16.43 | 45.11 | 64.48 |
| TGFBR2-site 5 | Sense strand | 14 | \ | \ | \ | 0.57 | 0.94 |
| TGFBR2-site 6 | Antisense strand | 16, 17 | 2.81 | 0.06 | 0.40 | 1.63 | 0.95 |
| TGFBR2-site 7 | Sense strand | 5 | 15.82 | 6.70 | 10.95 | 46.91 | 39.17 |
| TGFBR2-site 8 | Antisense strand | 4, 5 | 37.06 | 50.17 | 58.26 | 51.55 | 45.65 |
| TGFBR2-site 9 | Antisense strand | 13, 14 | 0.13 | 1.11 | 0.68 | 1.64 | 3.15 |
| TGFBR2-site 10 | Antisense strand | 12, 13 | 3.66 | 6.58 | 2.70 | 3.28 | 12.77 |
| TRAC-site1 | Sense strand | 14 | 0.39 | 0.11 | \ | 0.32 | 1.01 |
| TRAC-site2 | Sense strand | 4 | 1.61 | 0.28 | \ | 4.63 | 1.39 |
| TRAC-site3 | Sense strand | 3 | \ | 2.24 | \ | 15.97 | 7.06 |
| TRAC-site4 | Antisense strand | 8 | 55.99 | 5.61 | 11.00 | 69.06 | 43.02 |
| PD-1-site1 | Antisense strand | 9 and/or 10 | 1.28 | 43.23 | 1.71 | 28.25 | 27.23 |
| PD-1-site2 | Antisense strand | 8 and/or 9 | \ | 22.99 | \ | 36.87 | 35.94 |
| PD-1-site3 | Antisense strand | 12 and/or 13 | \ | \ | \ | 7.12 | 10.35 |
| PD-1-site4 | Antisense strand | 7 and/or 8 | \ | \ | \ | 35.4 | 36.2 |
| PD-1-site5 | Antisense strand | 6 and/or 7 | 10.99 | 2.00 | 5.70 | 48.18 | 49.68 |
| PD-1-site6 | Sense strand | 16 | 0.15 | 0.69 | 0.55 | 0.05 | 0.20 |
| PD-1-site7 | Sense strand | 12 | 0.47 | 3.96 | 3.00 | 8.00 | 17.62 |
| PD-1-site8 | Sense strand | 9 | 9.60 | 12.86 | 6.39 | 44.9 | 47.91 |
| PD-1-site9 | Sense strand | 4 | 16.51 | 6.14 | 16.86 | 43.90 | 25.78 |
| PD-1-site10 | Sense strand | 8 | 22.22 | 2.33 | 16.70 | 49.34 | 37.87 |
| PD-1-site11 | Sense strand | 10 | 1.62 | 14.32 | 6.11 | 3.39 | 27.38 |
| PD-1-site12 | Sense strand | 9 | 7.04 | 15.31 | 6.49 | 29.61 | 25.56 |
| PD-1-site13 | Antisense strand | 7 | 46.32 | 43.61 | 26.97 | 52.34 | 26.70 |
| PD-1-site14 | Sense strand | 3 | 8.25 | 67.68 | 44.58 | 55.57 | 38.81 |
| PD-1-site15 | Sense strand | 5 | 44.06 | 3.81 | 34.90 | 61.06 | 48.17 |
| PD-1-site16 | Sense strand | 8 | 43.59 | 3.91 | 25.29 | 57.45 | 43.41 |
| PD-1-site17 | Sense strand | 7 | 45.59 | 1.43 | 31.17 | 63.79 | 37.96 |

Table 1 showed the editing efficiency of the editing system suitable for each target site with high editing efficiency, from which the editing target sites with high editing efficiency were identified. For the *CISH* gene, the target sites corresponding to site1, site5, site8, and site10 exhibited better effects, with the editing efficiency reaching 47.46% to 71.26%. For the *PD-1* gene, the target sites corresponding to site14, site15, site16, and site17 exhibited better effects, with the editing efficiency reaching 57.45% to 67.68%. For the *FAS* gene, the target sites corresponding to site4, site7, site8, and site10 exhibited better effects, with the editing efficiency reaching 49.05% to 61.68%. For the *TGFBR2* gene, the target sites corresponding to site1, site4, site7, and site8 exhibited better effects, with the editing efficiency reaching 46.91% to 64.48%. For the *TRAC* gene, the target site corresponding to site4 exhibited a better effect, with the editing efficiency reaching 69.06%.

### Example 2. Screening of Deaminases

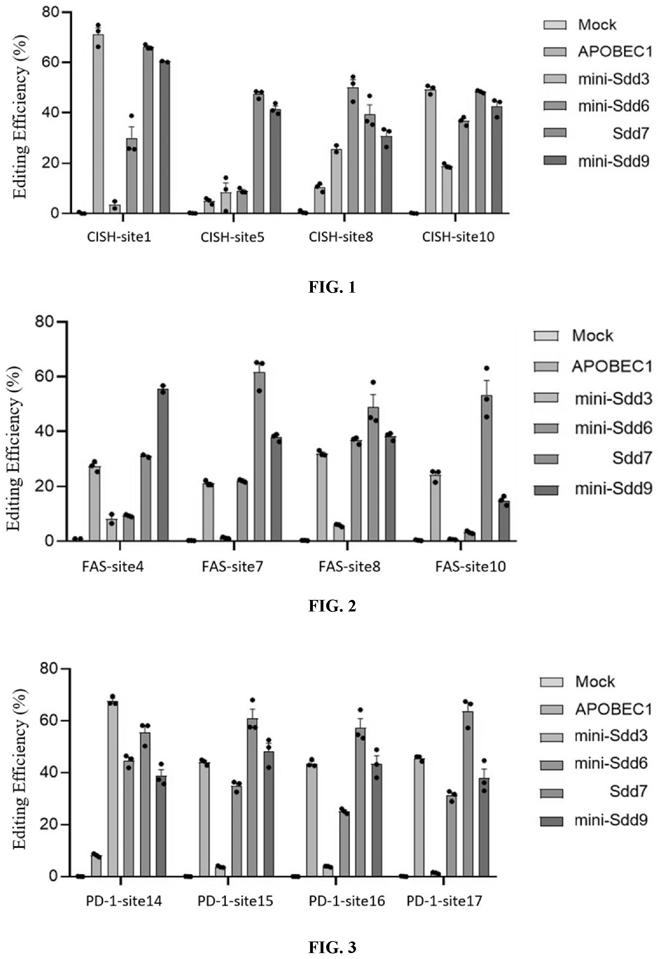

To construct a single-base editing system universally applicable to multiple genes, this example further validated and screened for deaminases effective in editing multiple target sites based on the preferred target sites identified in Example 1. The experimental results were shown in FIGS. 1 to 5. For the editing efficiency of each base editing system containing a different deaminase at preferred target sites as validated in FIGS. 1 to 5, the editing efficiency equal to or greater than 40% was defined as high-efficiency editing and marked as "+"; and the editing efficiency less than 40% was defined as non-high-efficiency editing and marked as "-". The statistical results were shown in Table 2. The data suggested that the base editors using APOBEC1, miniSdd3, or miniSdd6 as the deaminase did not achieve high-efficiency editing at certain preferred target sites in some target genes. Only the base editing systems using Sdd7 or miniSdd9 as the deaminase exhibited high-efficiency editing sites in all five genes, suggesting that they were ideal deaminases for the base editing system of the present disclosure.

**Table 2**

| CISH | | | | |
|---|---|---|---|---|
| | site1 | site5 | site8 | site10 |
| APOBEC1 | + | - | - | + |
| mini-Sdd3 | - | - | - | - |
| mini-Sdd6 | - | - | + | - |
| Sdd7 | + | + | - | + |
| mini-Sdd9 | + | + | - | + |

| FAS | | | | |
|---|---|---|---|---|
| | site4 | site7 | site8 | site10 |
| APOBEC1 | - | - | - | - |
| mini-Sdd3 | - | - | - | - |
| mini-Sdd6 | - | - | - | - |
| Sdd7 | - | + | + | + |
| mini-Sdd9 | + | - | + | - |

| PD-1 | | | | |
|---|---|---|---|---|
| | site14 | site 15 | site16 | site17 |
| APOBEC1 | - | + | + | + |
| mini-Sdd3 | + | - | - | - |
| mini-Sdd6 | + | - | - | - |
| Sdd7 | + | + | + | + |
| mini-Sdd9 | - | + | + | - |

| TGFBR2 | | | | |
|---|---|---|---|---|
| | site 1 | site4 | site7 | site8 |
| APOBEC1 | - | - | - | - |
| mini-Sdd3 | - | - | - | + |
| mini-Sdd6 | - | - | - | + |
| Sdd7 | + | + | + | + |
| mini-Sdd9 | + | + | + | + |

| TRAC | | | | |
|---|---|---|---|---|
| | site4 | | | |
| APOBEC1 | + | | | |
| mini-Sdd3 | - | | | |
| mini-Sdd6 | - | | | |
| Sdd7 | + | | | |
| mini-Sdd9 | + | | | |

### Example 3. Validation of Different Base Editing Systems

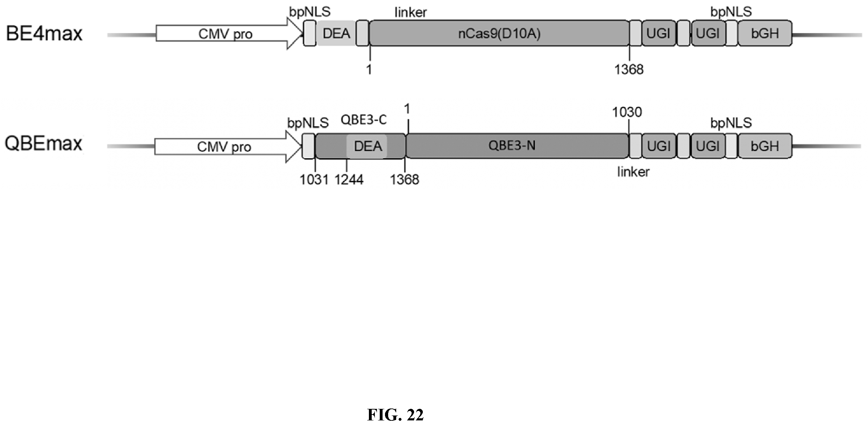

To verify the effects of different base editing polypeptides on the editing efficiency, this example selected, as the validation system, a QBEmax system in which a circularly permuted variant of a CRISPR protein was used as the DNA-binding protein. In this system, the amino acid sequence of the DNA-binding protein with the single-strand cleavage function was subjected to circular permutation (the linker for circular permutation was GGSGGSGGSGGSGGSGGSGG; SEQ ID NO: 90) to obtain a circularly permuted sequence having a length of 1388 amino acids (the circularly permuted sequence was as set forth in SEQ ID NO: 91, where the amino acid position of the circularly permuted sequence was defined in such a way that the methionine M encoded by a start codon was the first amino acid, and the glycine G directly linked to the first amino acid in the linker was the 1388^{th} amino acid). The circularly permuted sequence was cleaved at two sites to obtain a pair of separate polypeptides (SEQ ID NO: 92 and SEQ ID NO: 93). These separate polypeptides were the paired DNA-binding protein domains. Based on their positions at which they were fused to the deaminase, the domains linked to the N-terminus or C-terminus of the deaminase were defined as the deaminase N-terminal linker domain (QBE-N) and the deaminase C-terminal linker domain (QBE-C), respectively. The polypeptide of the QBEmax system comprised the following structure: [QBE-N]-[deaminase]-[QBE-C], where "-" was a linker. FIG. 22 was a schematic diagram of the structure of the base editing polypeptide in this example.

The two preferred deaminases Sdd7 and mini-Sdd9 screened in Example 2 were used as the deaminase domains of the base editing polypeptide to obtain four base editing polypeptides respectively: Sdd7-QBEmax (SEQ ID NO: 79), Sdd7-BE4max (SEQ ID NO: 77), mini-Sdd9-QBEmax (SEQ ID NO: 80), and mini-Sdd9-BE4max (SEQ ID NO: 75).

On the one hand, plasmids expressing the above editing polypeptides and the corresponding sgRNAs were transfected into HEK293T-cells. The editing efficiency at these target sites was statistically analyzed, and their performances at these CAR-T-related sites were compared in parallel.

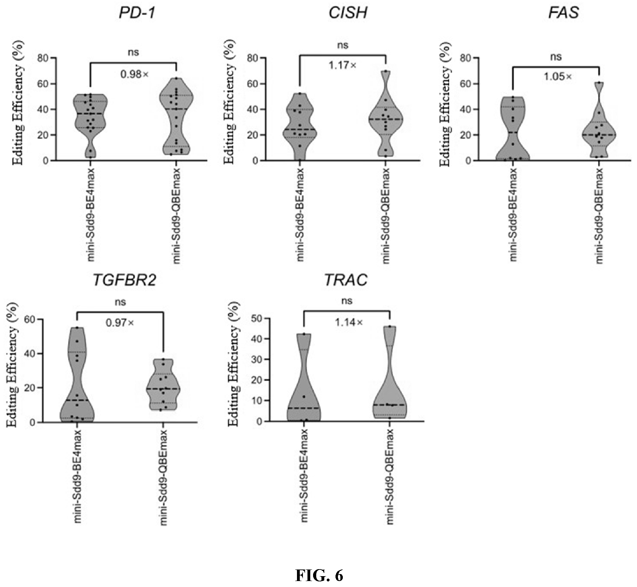

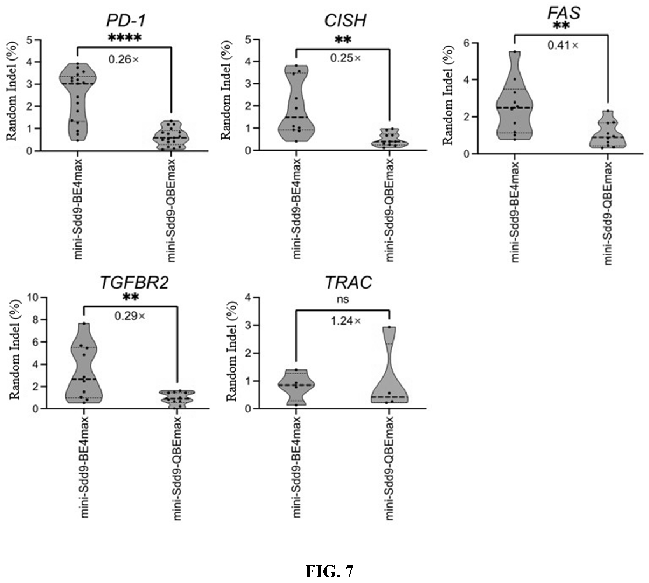

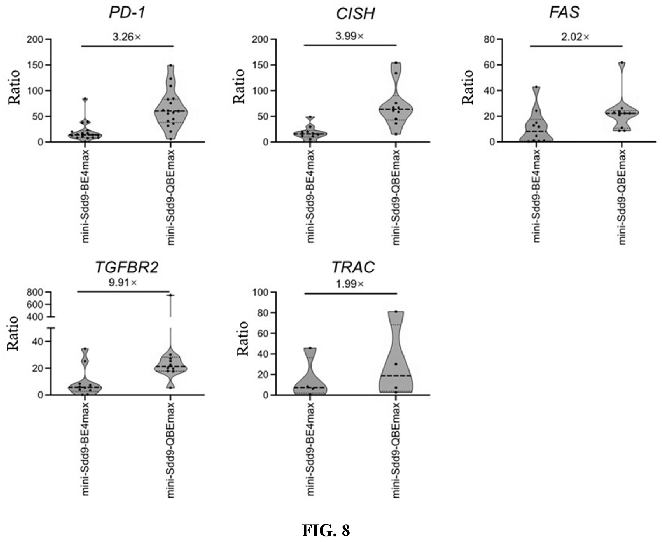

FIG. 6 showed that the average editing efficiency of mini-Sdd9-QBEmax in each of the five target genes was comparable to the overall average editing efficiency of mini-Sdd9-BE4max. FIG. 7 showed that the average indel of mini-Sdd9-QBEmax in each of the five target genes was significantly lower than the average indel of mini-Sdd9-BE4max. FIG. 8 showed the statistically analyzed editing efficiency/indel ratio and found that the editing efficiency/indel ratio of mini-Sdd9-QBEmax in each of the five target genes was significantly increased by 1.99- to 9.91-fold, as compared to mini-Sdd9-BE4max.

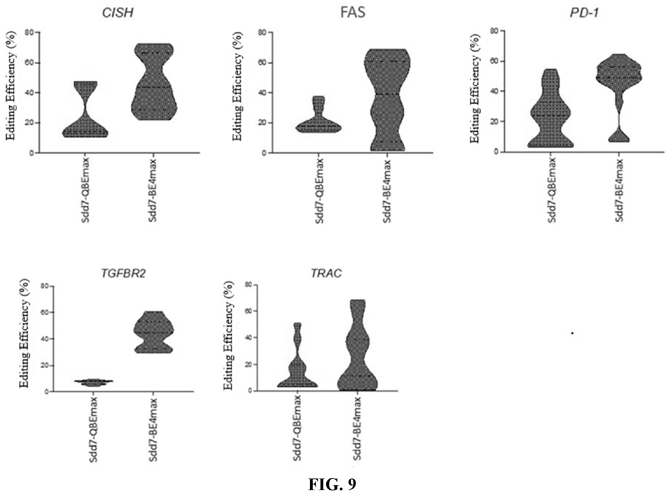

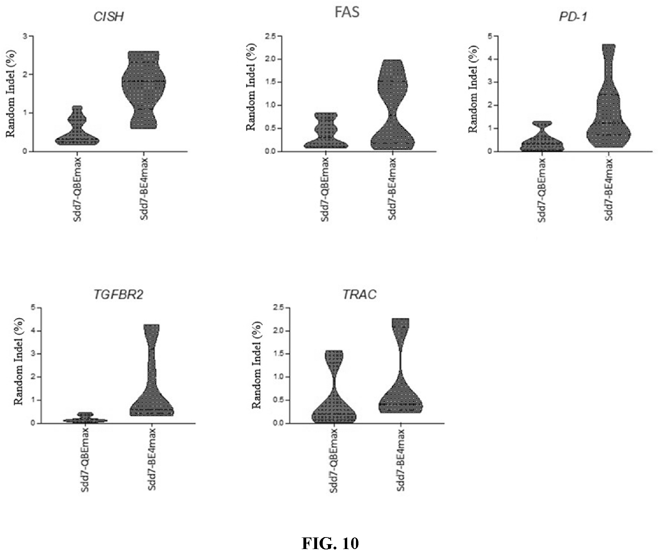

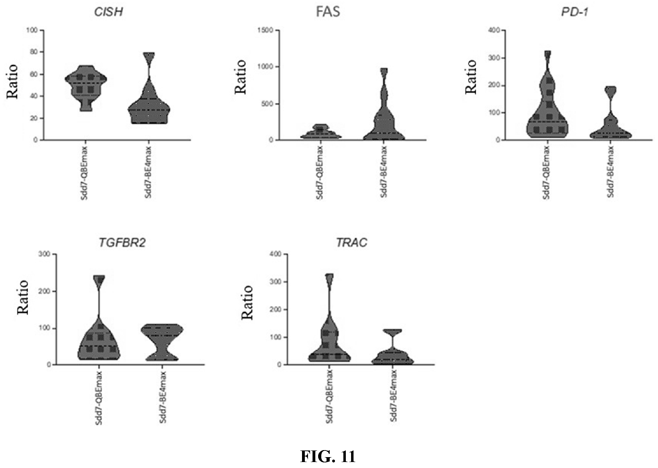

FIG. 9 showed that the average editing efficiency of Sdd7-QBEmax in each of the five target genes was slightly lower than that of Sdd7-BE4max. Meanwhile, FIG. 10 showed that the average indel of Sdd7-QBEmax was lower than that of Sdd7-BE4max. FIG. 11 showed the statistically analyzed editing efficiency/indel ratio, suggesting that the editing efficiency of Sdd7-QBEmax in the *CISH*, *PD-1* and *TRAC* was better than that of Sdd7-BE4max, while the editing efficiency of Sdd7-BE4max in the *FAS* and *TGFBR2* was better than that of Sdd7-QBEmax.

In summary, the QBEmax and BE4max base editing polypeptides obtained by fusing a mini-Sdd9 or Sdd7 deaminase to nCas9 or a circularly permuted variant protein of CRISPR exhibited comparable editing efficiency at the gene loci associated with enhancement of T-cells, each possessing its own advantages. Therefore, different editing systems could be adapted as appropriate depending on the deaminase selected.

### Example 4. Validation of Editing Purity at Target Sites

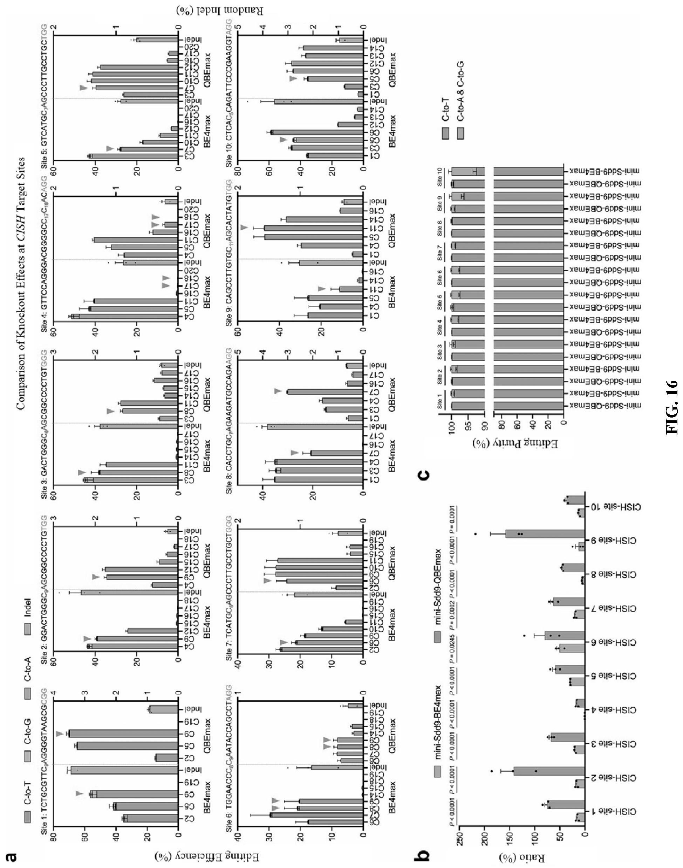

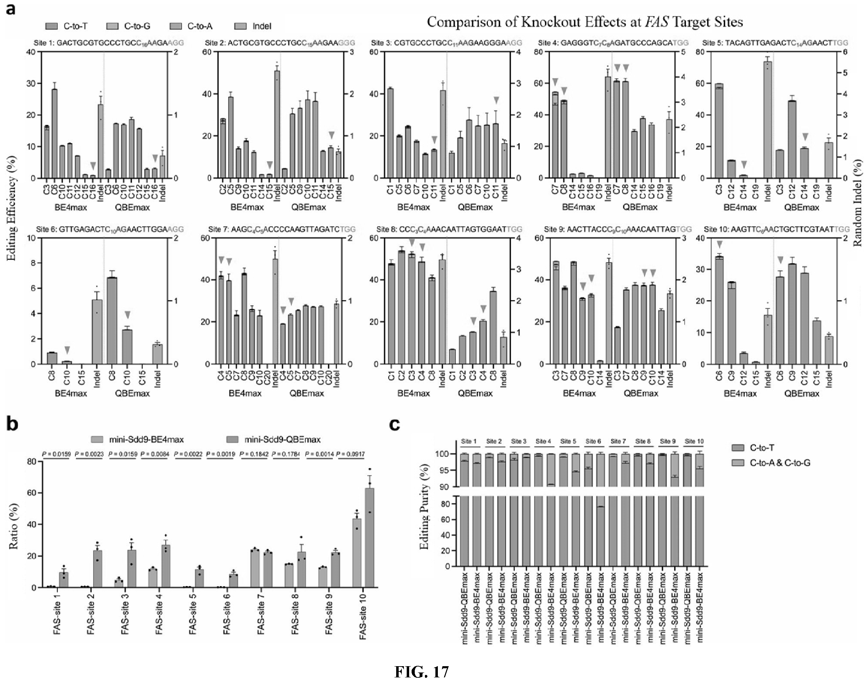

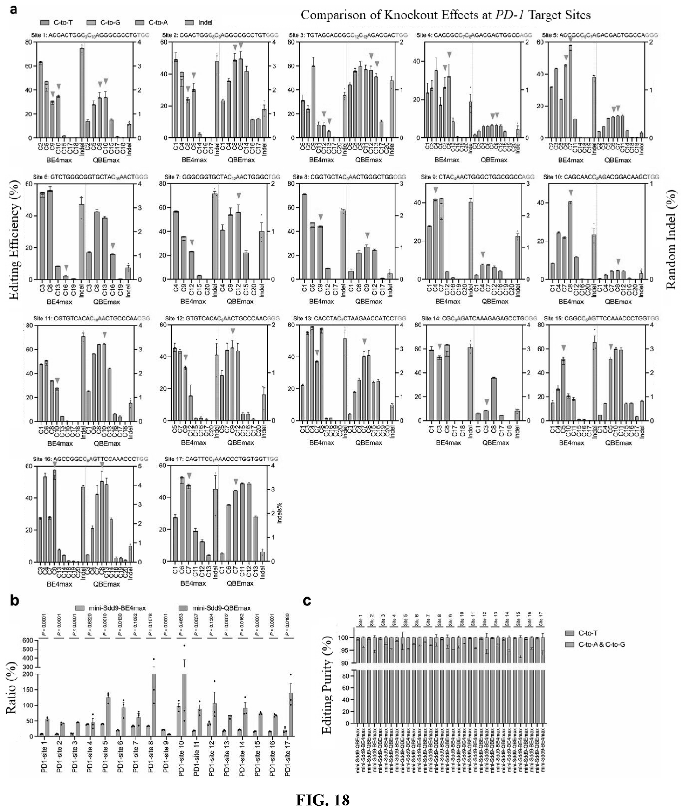

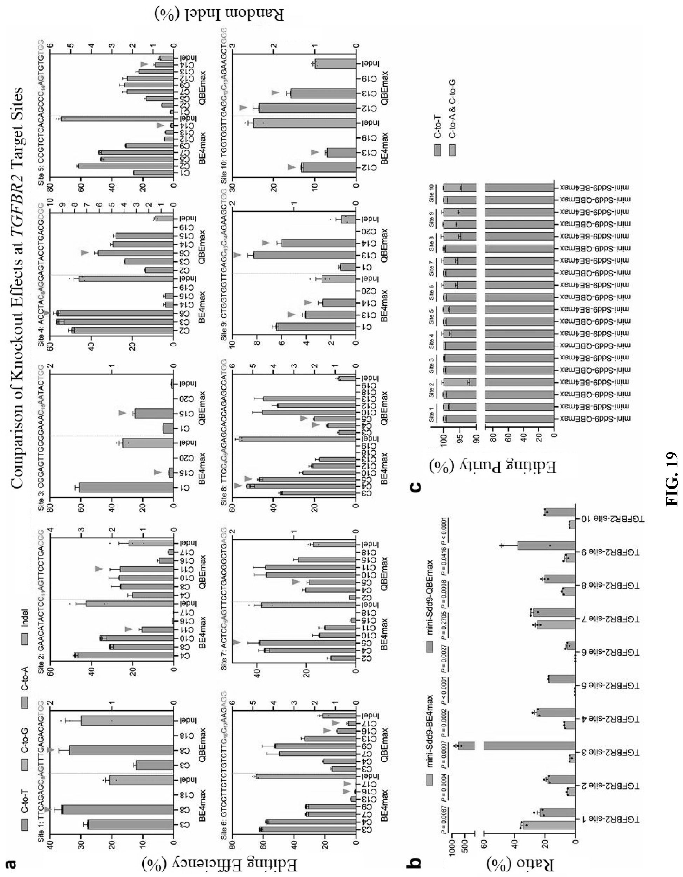

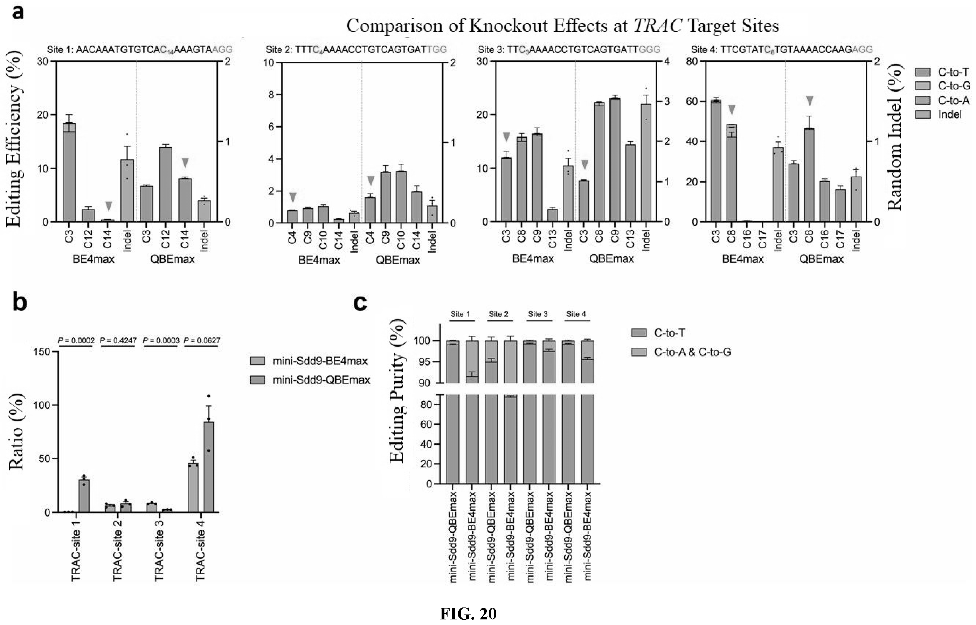

In this example, the mini-Sdd9 deaminase was linked to different base editing polypeptide scaffolds to validate the editing purity at each of the sites in the five genes described above. Accidental indels and impure base editing by-products (*e.g.*, C-to-G and C-to-A) were still frequently observed when performing base editing (C-to-T) using common BE tools. Formation of impure edits might introduce a new missense mutation into the target gene that was originally intended to be knocked out. Therefore, an ideal BE tool should possess not only high editing efficiency but also high editing purity to ensure safety. FIGS. 16-20 showed the editing efficiency and purity of mini-Sdd9-QBEmax and mini-Sdd9-BE4max for knocking out the above five genes (51 target sites). In the above figures, a showed the editing efficiency and indels at different sites (the position indicated by the blue arrow was the desired mutated base); b showed the editing efficiency/indel ratios at different sites; c showed the proportion of C-to-T at each edited site (C-to-T/C-to-R, R was A or G), namely, the editing purity. The analysis and statistics of FIGS. 16-20 were shown in Table 4. The editing efficiency equal to or greater than 40% was marked as "+" and the editing efficiency less than 40% was marked as "-"; the editing purity equal to or greater than 95% was marked as "+", and the editing purity less than 95% was marked as "-". The mark "+" for both the efficiency and purity indicated that the editing system exhibited a good comprehensive effect at a given site.

As could be appreciated from the comprehensive analysis of the editing efficiency and editing purity in FIGS. 16-20 and Table 3, for the *CISH* gene, mini-Sdd9-BE4max showed the best effect at site1, and mini-Sdd9-QBEmax showed the best effect at site1 and site9; for the *FAS* gene, mini-Sdd9-BE4max showed the best effect at site7, and mini-Sdd9-QBEmax showed the best effect at site4 and site8; for the *PD-1* gene, mini-Sdd9-BE4max showed the highest editing efficiency at site17 and a better comprehensive effect at site5 and site8, and mini-Sdd9-QBEmax exhibited better effects at all of site2, site3, site7, site11, site12, site15, site16, and site17; for the *TGFBR2* gene, mini-Sdd9-BE4max showed the best editing efficiency at site8 and the best comprehensive effect at site4, and mini-Sdd9-QBEmax showed higher editing purity at all sites in this gene, but moderate editing efficiency; for the *TRAC* gene, both mini-Sdd9-BE4max and mini-Sdd9-QBEmax showed a better effect at site4.

In summary, by including the editing purity into the screening criteria, the 51 target sites were subjected to a secondary screening. It was found that QBEmax and BE4max showed some differences at the preferred editing sites in the five target genes. Therefore, different editing polypeptides could be adapted as appropriate during actual gene knockout. Furthermore, some preferred target sites (*e.g.,* site9 in the *CISH* gene; site2, site3, site7, site11, and site12 in the *PD-1* gene) for QBEmax obtained in this example were supplemented as candidate target sites for subsequent simultaneous knockout experiments.

**Table 3**

| CISH | | | | | | | | | | | |
|---|---|---|---|---|---|---|---|---|---|---|---|
| | site | 1 | 2 | 3 | 4 | 5 | 6 | 7 | 8 | 9 | 10 |
| BE4max | Efficiency | + | - | - | - | - | - | - | - | - | + |
| | Purity | + | + | + | + | + | + | + | + | + | - |
| QBEmax | Efficiency | + | - | - | - | - | - | - | - | + | - |
| | Purity | + | + | + | + | + | + | + | + | + | + |

| FAS | | | | | | | | | | | |
|---|---|---|---|---|---|---|---|---|---|---|---|
| | site | 1 | 2 | 3 | 4 | 5 | 6 | 7 | 8 | 9 | 10 |
| BE4max | Efficiency | - | - | - | + | - | - | + | - | - | - |
| | Purity | + | + | + | - | - | - | + | + | - | - |
| QBEmax | Efficiency | - | - | - | + | - | - | - | + | - | - |
| | Purity | + | + | + | + | + | - | + | + | + | + |

| PD-1 | | | | | | | | | | | |
|---|---|---|---|---|---|---|---|---|---|---|---|
| | site | 1 | 2 | 3 | 4 | 5 | 6 | 7 | 8 | 9 | 10 |
| BE4max | Efficiency | - | - | - | - | + | - | - | + | + | - |
| | Purity | + | - | + | + | + | + | + | + | - | + |
| QBEmax | Efficiency | - | + | + | - | - | - | + | - | - | - |
| | Purity | + | + | + | + | + | + | + | + | + | + |
| | site | 11 | 12 | 13 | 14 | 15 | 16 | 17 | | | |
| BE4max | Efficiency | - | - | - | + | + | + | + | | | |
| | Purity | + | - | + | - | - | - | - | | | |
| QBEmax | Efficiency | + | + | - | - | + | + | + | | | |
| | Purity | + | + | + | + | + | + | + | | | |

| TGFBR2 | | | | | | | | | | | |
|---|---|---|---|---|---|---|---|---|---|---|---|
| | site | 1 | 2 | 3 | 4 | 5 | 6 | 7 | 8 | 9 | 10 |
| BE4max | Efficiency | - | - | - | + | - | - | - | + | - | - |
| | Purity | + | - | + | + | + | + | + | - | - | - |
| QBEmax | Efficiency | - | - | - | - | - | - | - | - | - | - |
| | Purity | + | + | + | + | + | + | + | + | + | + |

| TRAC | | | | | | | | | | | |
|---|---|---|---|---|---|---|---|---|---|---|---|
| | site | 1 | 2 | 3 | 4 | | | | | | |
| BE4max | Efficiency | - | - | - | + | | | | | | |
| | Purity | - | - | + | + | | | | | | |
| QBEmax | Efficiency | - | - | - | + | | | | | | |
| | Purity | + | - | + | + | | | | | | |

### Example 5. Simultaneous Knockout of Five Genes

In Examples 1-4, the inventors stepwise screened for preferred target sites in the five genes, preferred deaminases, and preferred base editing polypeptides. In this example, the inventors permuted and combined the above parameters to obtain the combinations shown in Table 4 to validate the experimental effects of simultaneous knockout of multiple genes. The base editing polypeptides were co-transfected with five different sgRNAs into HEK293T cells to achieve simultaneous knockout of the five genes.

**Table 4**

| | Base Editing Polypeptide | Protospacer in Gene |
|---|---|---|
| Combination 1 | Sdd7-BE4max (SEQ ID NO: 77) | CISH-site1 (SEQ ID NO: 1) |
| | | FAS-site4 (SEQ ID NO: 14) |
| | | PD-1-site17 (SEQ ID NO: 47) |
| | | TGFBR2-site8 (SEQ ID NO: 28) |
| | | TRAC-site4 (SEQ ID NO: 51) |
| Combination 2 | Sdd7-QBEmax (SEQ ID NO: 79) | CISH-site1 (SEQ ID NO: 1) |
| | | FAS-site4 (SEQ ID NO: 14) |
| | | PD-1-site11 (SEQ ID NO: 41) |
| | | TGFBR2-site4 (SEQ ID NO: 24) |
| | | TRAC-site4 (SEQ ID NO: 51) |
| Combination 3 | mini-Sdd9-BE4max (EQ ID NO: 75) | CISH-site1 (SEQ ID NO: 1) |
| | | FAS-site4 (SEQ ID NO: 14) |
| | | PD-1-site11 (SEQ ID NO: 41) |
| | | TGFBR2-site4 (SEQ ID NO: 24) |
| | | TRAC-site4 (SEQ ID NO: 51) |
| Combination 4 | mini-Sdd9-QBEmax (SEQ ID NO: 80) | CISH-site1 (SEQ ID NO: 1) |
| | | FAS-site4 (SEQ ID NO: 14) |
| | | PD-1-site11 (SEQ ID NO: 41) |
| | | TGFBR2-site4 (SEQ ID NO: 24) |
| | | TRAC-site4 (SEQ ID NO: 51) |

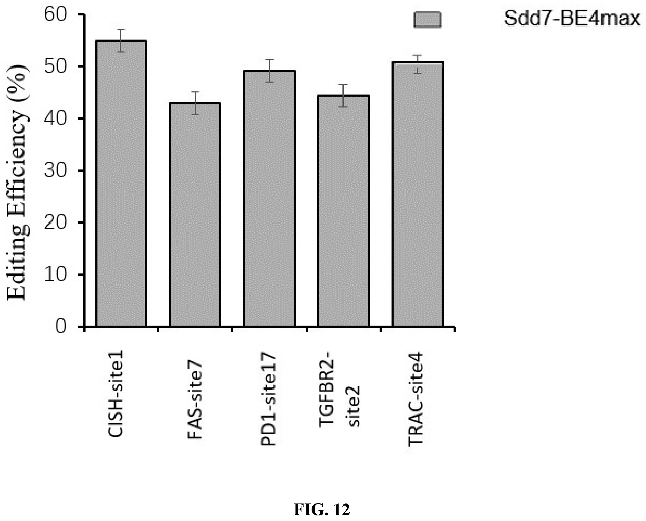

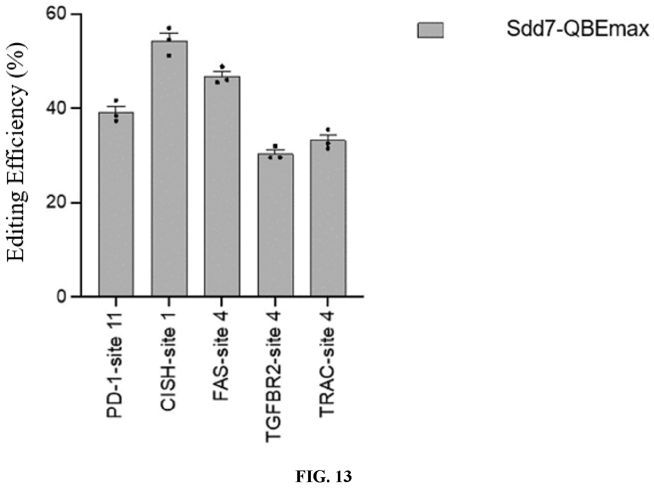

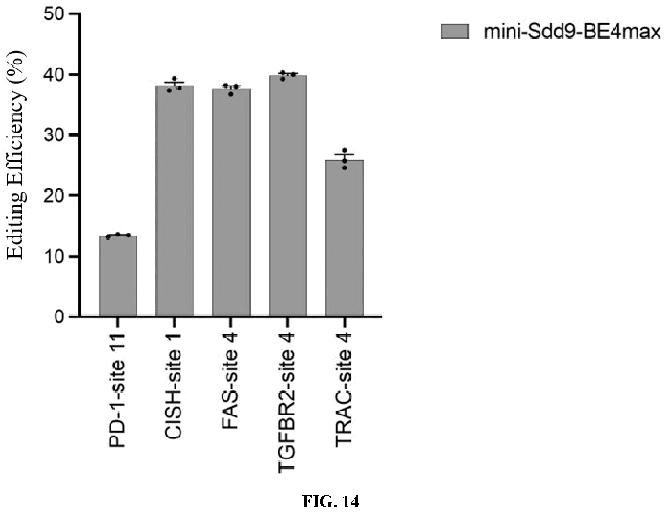

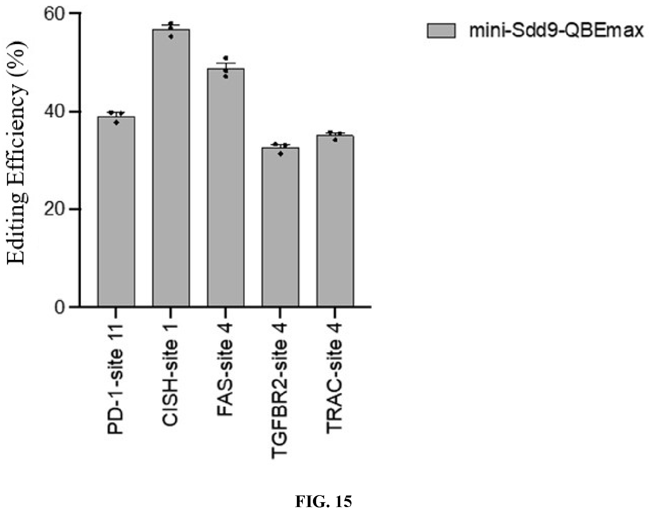

FIG. 12 showed the editing efficiency of Combination 1, which was relatively stable and consistently high, ranging from 42.9% to 54.88%. FIG. 13 showed the editing efficiency of Combination 2, with relatively high editing efficiency of 54.21% for the *CISH* gene and a comprehensive editing efficiency ranging from 30.35% to 54.21% for Combination 2. FIG. 14 showed the editing efficiency of Combination 3, suggesting that this system had a relatively weak ability to edit *PD-1* and *TRAC*, but consistently good ability to edit *CISH*, *FAS*, and *TGFBR2*, ranging between 37.66% and 39.86%. FIG. 15 showed the editing efficiency of Combination 4, with the overall editing ability at a relatively high level and the editing efficiency ranging between 32.65% and 56.78%. Therefore, the technical solutions of the present application could modify the genes associated with T-cell function enhancement.

While the present disclosure has been described with reference to specific embodiments herein, those skilled in the art will appreciate that various variations and equivalent substitutions may be made without departing from the true spirits and scopes of the present disclosure. In addition, various modifications may be made to adapt a specific circumstance, material, composition of substance, process, process step or steps to the purposes, spirits and scopes of the present disclosure. All such modifications are intended to be within the scope of the appended claims.

Nucleotide or amino acid sequences involved herein:
>SEQ ID NO: 1 CISH-site1
   TCTGCGTTCAGGGGTAAGCG
>SEQ ID NO: 2 CISH-site2
   GGACTGGGCAGCGGCCCCTG
>SEQ ID NO: 3 CISH-site3
   GACTGGGCAGCGGCCCCTGT
>SEQ ID NO: 4 CISH-site4
   GTTCCAGGGACGGGGCCCAC
>SEQ ID NO: 5 CISH-site5
   GTCATGCAGCCCTTGCCTGC
>SEQ ID NO: 6 CISH-site6
   TGGAACCCCAATACCAGCCT
>SEQ ID NO: 7 CISH-site7
   TCATGCAGCCCTTGCCTGCT
>SEQ ID NO: 8 CISH-site8
   CACCTGCAGAAGATGCCAGA
>SEQ ID NO: 9 CISH-site9
   CAGCCTTGTGCAGCACTATG
>SEQ ID NO: 10 CISH-site10
   CTCACCAGATTCCCGAAGGT
>SEQ ID NO: 11 FAS-site1
   GACTGCGTGCCCTGCCAAGA
>SEQ ID NO: 12 FAS-site2
   ACTGCGTGCCCTGCCAAGAA
>SEQ ID NO: 13 FAS-site3
   CGTGCCCTGCCAAGAAGGGA
>SEQ ID NO: 14 FAS-site4
   GAGGGTCCAGATGCCCAGCA
>SEQ ID NO: 15 FAS-site5
   TACAGTTGAGACTCAGAACT
>SEQ ID NO: 16 FAS-site6
   GTTGAGACTCAGAACTTGGA
>SEQ ID NO: 17 FAS-site7
   AAGCCACCCCAAGTTAGATC
>SEQ ID NO: 18 FAS-site8
   CCCCAAACAATTAGTGGAAT
>SEQ ID NO: 19 FAS-site9
   AACTTACCCCAAACAATTAG
>SEQ ID NO: 20 FAS-site10
   AAGTTCAACTGCTTCGTAAT
>SEQ ID NO: 21 TGFBR2-site1
   TTCAGAGCAGTTTGAGACAG
>SEQ ID NO: 22 TGFBR2-site2
   GAACATACTCCAGTTCCTGA
>SEQ ID NO: 23 TGFBR2-site3
   CGGAGTTGGGGAAACAATAC
>SEQ ID NO: 24 TGFBR2-site4
   ACCTACAGGAGTACCTGACG
>SEQ ID NO: 25 TGFBR2-site5
   CCGTCTCACAGCCCAGTGTG
>SEQ ID NO: 26 TGFBR2-site6
   GTCCTTCTCTGTCTTCCAAG
>SEQ ID NO: 27 TGFBR2-site7
   ACTCCAGTTCCTGACGGCTG
>SEQ ID NO: 28 TGFBR2-site8
   TTCCCAGAGCACCAGAGCCA
>SEQ ID NO: 29 TGFBR2-site9
   CTGGTGGTTGAGCCAGAAGC
>SEQ ID NO: 30 GFbR2-site10
   TGGTGGTTGAGCCAGAAGCT
>SEQ ID NO: 31 PD-1-site1
   ACGACTGGCCAGGGCGCCTG
>SEQ ID NO: 32 PD-1-site2
   CGACTGGCCAGGGCGCCTGT
>SEQ ID NO: 33 PD-1-site3
   TGTAGCACCGCCCAGACGAC
>SEQ ID NO: 34 PD-1-site4
   CACCGCCCAGACGACTGGCC
>SEQ ID NO: 35 PD-1-site5
   ACCGCCCAGACGACTGGCCA
>SEQ ID NO: 36 PD-1-site6
   GTCTGGGCGGTGCTACAACT
>SEQ ID NO: 37 PD-1-site7
   GGGCGGTGCTACAACTGGGC
>SEQ ID NO: 38 PD-1-site8
   CGGTGCTACAACTGGGCTGG
>SEQ ID NO: 39PD-1-site9
   CTACAACTGGGCTGGCGGCC
>SEQ ID NO: 40 PD-1-site10
   CAGCAACCAGACGGACAAGC
>SEQ ID NO: 41 PD-1-site11
   CGTGTCACACAACTGCCCAA
>SEQ ID NO: 42 PD-1-site12
   GTGTCACACAACTGCCCAAC
>SEQ ID NO: 43 PD-1-site13
   CACCTACCTAAGAACCATCC
>SEQ ID NO: 44 PD-1-site14
   CGCAGATCAAAGAGAGCCTG
>SEQ ID NO: 45 PD-1-site15
   CGGCCAGTTCCAAACCCTGG
>SEQ ID NO: 46 PD-1-site16
   AGCCGGCCAGTTCCAAACCC
>SEQ ID NO: 47 PD-1-site17
   CAGTTCCAAACCCTGGTGGT
>SEQ ID NO: 48 TRAC-site1
   AACAAATGTGTCACAAAGTA
>SEQ ID NO: 49 TRAC-site2
   TTTCAAAACCTGTCAGTGAT
>SEQ ID NO: 50 TRAC-site3
   TTCAAAACCTGTCAGTGATT
>SEQ ID NO: 51 TRAC-site4
   TTCGTATCTGTAAAACCAAG
>SEQ ID NO: 52 NLS
   KKRKV
>SEQ ID NO: 53 NLS
   PKKKRKV
>SEQ ID NO: 54 NLS
   KRPAATKKAGQAKKKK
>SEQ ID NO: 55 NLS
   MDSLLMNRRKFLYQFKNVRWAKGRRETYLC
>SEQ ID NO: 56 NLS
   KRTADGSEFEPKKKRKV
>SEQ ID NO: 57 NLS
   PAAKRVKLD
>SEQ ID NO: 58 NLS
   RQRRNELKRSP
>SEQ ID NO: 59 NLS
   NQSSNFGPMKGGNFGGRSSGPYGGGGQYFAKPRNQGGY
>SEQ ID NO: 60 NLS
   RMRIZFKNKGKDTAELRRRRVEVSVELRKAKKDEQILKRRNV
>SEQ ID NO: 61 NLS
   VSRKRPRP
>SEQ ID NO: 62 NLS
   PPKKARED
>SEQ ID NO: 63 NLS
   POPKKKPL
>SEQ ID NO: 64 NLS
   SALIKKKKKMAP
>SEQ ID NO: 65 NLS
   RKLKKKIKKL
>SEQ ID NO: 66 NLS
   REKKKFLKRR
>SEQ ID NO: 67 NLS
   KRKGDEVDGVDEVAKKKSKK
>SEQ ID NO: 68 NLS
   RKCLQAGMNLEARKTKK
>SEQ ID NO: 69 deaminase rAPOBEC1: 
SSETGPVAVDPTLRRRIEPHEFEVFFDPRELRKETCLLYEINWGGRHSIWRHTSQNT
NKHVEVNFIEKFTTERYFCPNTRCSITWFLSWSPCGECSRAITEFLSRYPHVTLFIYIARL
YHHADPRNRQGLRDLISSGVTIQIMTEQESGYCWRNFVNYSPSNEAHWPRYPHLWVR
LYVLELYCIILGLPPCLNILRRKQPQLTFFTIALQSCHYQRLPPHILWATGLK
>SEQ ID NO: 70 mini-Sdd3 
PRRFNPACWQGLKPYDQAGTAEGNLFIAPGKRWNTRPMQASKLEVGPQSDLHPQ
WRSRKAPWHIEGKIAAYMRQKGFTDGCVYLNARPCSGPDGCARNLPDLLPVGSTLHV
HARYIDRTGETRFYYREYRGTGKALT
>SEQ ID NO: 71 mini-Sdd6 
PASKPSRFKPMRTDPAKADEIRPHVGKDRAVATLWDADGNRVLGLHSADDDGPAA
TAAWKPPWRDYVRLRRHVEAHAAARMHQDGHKTMVMYINLPPCKYFDGCKLNLED
ILPKGSTLWMHRVFQNGGTKIYQFNGTGRAYV
>SEQ ID NO: 72 mini-Sdd7 
EGGGPGAVPEGGDGPPAVPAEEVERLRGELPPPVVPGTGQKTHGRWIGPDGRVRAI
VSGRDEDAALVHAQLAAKGIPDEPTRNSDVEQKLAAHMVANGIRHVTLVINHRPCRGF
DDSCDTLVPIILPEGCTLTVHGQTDKGMRVRVRYTGGARPWWS
>SEQ ID NO: 73 mini-Sdd9 
CPIWVKNALQELVGRKETSGKVFDVDGKPIGPDIQSGYKDRELRRGVYETLRKSP
FFQKHFPSTATWYVSLHVEAQYAVWMLRNRIKHATVVINNTYVCSDMNRLHDNCMTA
VPHILPEGYTMTVWKADRTEVTLRGKAPKE
>SEQ ID NO: 74 rAPOBEC1-BE4max 
MKRTADGSEFESPKKKRKVSSETGPVAVDPTLRRRIEPHEFEVFFDPRELRKETCLL
YEINWGGRHSIWRHTSQNTNKHVEVNFIEKFTTERYFCPNTRCSITWFLSWSPCGECSR
AITEFLSRYPHVTLFIYIARLYHHADPRNRQGLRDLISSGVTIQIMTEQESGYCWRNFVN
YSPSNEAHWPRYPHLWVRLYVLELYCIILGLPPCLNILRRKQPQLTFFTIALQSCHYQRL
PPHILWATGLKSGGSSGGSSGSETPGTSESATPESSGGSSGGSDKKYSIGLAIGTNSVGW
AVITDEYKVPSKKFKVLGNTDRHSIKKNLIGALLFDSGETAEATRLKRTARRRYTRRKN
RICYLQEIFSNEMAKVDDSFFIIRLEESFLVEEDKKIIERIIPIFGNIVDEVAYIIEKYPTIYII
LRKKLVDSTDKADLRLIYLALAHMIKFRGHFLIEGDLNPDNSDVDKLFIQLVQTYNQLF
EENPINASGVDAKAILSARLSKSRRLENLIAQLPGEKKNGLFGNLIALSLGLTPNFKSNF
DLAEDAKLQLSKDTYDDDLDNLLAQIGDQYADLFLAAKNLSDAILLSDILRVNTEITKA
PLSASMIKRYDEHHQDLTLLKALVRQQLPEKYKEIFFDQSKNGYAGYIDGGASQEEFY
KFIKPILEKMDGTEELLVKLNREDLLRKQRTFDNGSIPHQIHLGELHAILRRQEDFYPFL
 
KDNREKIEKILTFRIPYYVGPLARGNSRFAWMTRKSEETITPWNFEEVVDKGASAQSFIE
RMTNFDKNLPNEKVLPKHSLLYEYFTVYNELTKVKYVTEGMRKPAFLSGEQKKAIVD
LLFKTNRKVTVKQLKEDYFKKIECFDSVEISGVEDRFNASLGTYHDLLKIIKDKDFLDN
EENEDILEDIVLTLTLFEDREMIEERLKTYAHLFDDKVMKQLKRRRYTGWGRLSRKLIN
GIRDKQSGKTILDFLKSDGFANRNFMQLIHDDSLTFKEDIQKAQVSGQGDSLHEHIANL
AGSPAIKKGILQTVKVVDELVKVMGRHKPENIVIEMARENQTTQKGQKNSRERMKRIE
EGIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDNL
TKAERGGLSELDKAGFIKRQLVETRQITKHVAQILDSRMNTKYDENDKLIREVKVITLK
SKLVSDFRKDFQFYKVREINNYHHAHDAYLNAVVGTALIKKYPKLESEFVYGDYKVY
DVRKMIAKSEQEIGKATAKYFFYSNIMNFFKTEITLANGEIRKRPLIETNGETGEIVWDK
GRDFATVRKVLSMPQVNIVKKTEVQTGGFSKESILPKRNSDKLIARKKDWDPKKYGGF
DSPTVAYSVLVVAKVEKGKSKKLKSVKELLGITIMERSSFEKNPIDFLEAKGYKEVKKD
LIIKLPKYSLFELENGRKRMLASAGELQKGNELALPSKYVNFLYLASHYEKLKGSPEDN
EQKQLFVEQHKHYLDEIIEQISEFSKRVILADANLDKVLSAYNKHRDKPIREQAENIIHL
FTLTNLGAPAAFKYFDTTIDRKRYTSTKEVLDATLIHQSITGLYETRIDLSQLGGDSGGS
GGSGGSTNLSDIIEKETGKQLVIQESILMLPEEVEEVIGNKPESDILVHTAYDESTDENVM
LLTSDAPEYKPWALVIQDSNGENKIKMLSGGSGGSGGSTNLSDIIEKETGKQLVIQESIL
MLPEEVEEVIGNKPESDILVHTAYDESTDENVMLLTSDAPEYKPWALVIQDSNGENKIK
MLSGGSKRTADGSEFEPKKKRKV
>SEQ ID NO: 75 mini-Sdd9-BE4max 
MKRTADGSEFESPKKKRKVCPIWVKNALQELVGRKETSGKVFDVDGKPIGPDIQS
GYKDRELRRGVYETLRKSPFFQKHFPSTATWYVSLHVEAQYAVWMLRNRIKHATVVI
NNTYVCSDMNRLHDNCMTAVPHILPEGYTMTVWKADRTEVTLRGKAPKESGGSSGG
SSGSETPGTSESATPESSGGSSGGSDKKYSIGLAIGTNSVGWAVITDEYKVPSKKFKVLG
NTDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRLIY
LALAHMIKFRGHFLIEGDLNPDNSDVDKLFIQLVQTYNQLFEENPINASGVDAKAILSA
RLSKSRRLENLIAQLPGEKKNGLFGNLIALSLGLTPNFKSNFDLAEDAKLQLSKDTYDD
DLDNLLAQIGDQYADLFLAAKNLSDAILLSDILRVNTEITKAPLSASMIKRYDEHHQDL
TLLKALVRQQLPEKYKEIFFDQSKNGYAGYIDGGASQEEFYKFIKPILEKMDGTEELLV
KLNREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLPK
HSLLYEYFTVYNELTKVKYVTEGMRKPAFLSGEQKKAIVDLLFKTNRKVTVKQLKED
 
YFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSD
GFANRNFMQLIHDDSLTFKEDIQKAQVSGQGDSLHEHIANLAGSPAIKKGILQTVKVVD
ELVKVMGRHKPENIVIEMARENQTTQKGQKNSRERMKRIEEGIKELGSQILKEHPVEN
TQLQNEKLYLYYLQNGRDMYVDQELDINRLSDYDVDHIVPQSFLKDDSIDNKVLTRSD
KNRGKSDNVPSEEVVKKMKNYWRQLLNAKLITQRKFDNLTKAERGGLSELDKAGFIK
RQLVETRQITKHVAQILDSRMNTKYDENDKLIREVKVITLKSKLVSDFRKDFQFYKVRE
INNYHHAHDAYLNAVVGTALIKKYPKLESEFVYGDYKVYDVRKMIAKSEQEIGKATA
KYFFYSNIMNFFKTEITLANGEIRKRPLIETNGETGEIVWDKGRDFATVRKVLSMPQVNI
VKKTEVQTGGFSKESILPKRNSDKLIARKKDWDPKKYGGFDSPTVAYSVLVVAKVEKG
KSKKLKSVKELLGITIMERSSFEKNPIDFLEAKGYKEVKKDLIIKLPKYSLFELENGRKR
MLASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDSGGSGGSGGSTNLSDIIEKETGK
QLVIQESILMLPEEVEEVIGNKPESDILVHTAYDESTDENVMLLTSDAPEYKPWALVIQD
SNGENKIKMLSGGSGGSGGSTNLSDIIEKETGKQLVIQESILMLPEEVEEVIGNKPESDIL
VHTAYDESTDENVMLLTSDAPEYKPWALVIQDSNGENKIKMLSGGSKRTADGSEFEPK
KKRKV
>SEQ ID NO: 76 mini-Sdd6-BE4max 
MKRTADGSEFESPKKKRKVPASKPSRFKPMRTDPAKADEIRPHVGKDRAVATLWD
ADGNRVLGLHSADDDGPAATAAWKPPWRDYVRLRRHVEAHAARMHQDGHKTMV
MYINLPPCKYFDGCKLNLEDILPKGSTLWMHRVFQNGGTKIYQFNGTGRAYVSGGSSG
GSSGSETPGTSESATPESSGGSSGGSDKKYSIGLAIGTNSVGWAVITDEYKVPSKKFKVL
GNTDRHSIKKNLIGALLFDSGETAEATRLKRTARRRYTRRKNRICYLQEIFSNEMAKVD
DSFFHRLEESFLVEEDKKHERHPIFGNIVDEVAYHEKYPTIYHLRKKLVDSTDKADLRLI
YLALAHMIKFRGHFLIEGDLNPDNSDVDKLFIQLVQTYNQLFEENPINASGVDAKAILS
ARLSKSRRLENLIAQLPGEKKNGLFGNLIALSLGLTPNFKSNFDLAEDAKLQLSKDTYD
DDLDNLLAQIGDQYADLFLAAKNLSDAILLSDILRVNTEITKAPLSASMIKRYDEHHQD
LTLLKALVRQQLPEKYKEIFFDQSKNGYAGYIDGGASQEEFYKFIKPILEKMDGTEELL
VKLNREDLLRKQRTFDNGSIPHQIHLGELHAILRRQEDFYPFLKDNREKIEKILTFRIPYY
VGPLARGNSRFAWMTRKSEETITPWNFEEVVDKGASAQSFIERMTNFDKNLPNEKVLP
KHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFE
DREMIEERLKTYAHLFDDKVMKQLKRRRYTGWGRLSRKLINGIRDKQSGKTILDFLKS
 
DGFANRNFMQLIHDDSLTFKEDIQKAQVSGQGDSLHEHIANLAGSPAIKKGILQTVKVV
DELVKVMGRHKPENIVIEMARENQTTQKGQKNSRERMKRIEEGIKELGSQILKEHPVE
NTQLQNEKLYLYYLQNGRDMYVDQELDINRLSDYDVDHIVPQSFLKDDSIDNKVLTRS
DKNRGKSDNVPSEEVVKKMKNYWRQLLNAKLITQRKFDNLTKAERGGLSELDKAGFI
KRQLVETRQITKHVAQILDSRMNTKYDENDKLIREVKVITLKSKLVSDFRKDFQFYKVR
EINNYHHAHDAYLNAVVGTALIKKYPKLESEFVYGDYKVYDVRKMIAKSEQEIGKATA
KYFFYSNIMNFFKTEITLANGEIRKRPLIETNGETGEIVWDKGRDFATVRKVLSMPQVNI
VKKTEVQTGGFSKESILPKRNSDKLIARKKDWDPKKYGGFDSPTVAYSVLVVAKVEKG
KSKKLKSVKELLGITIMERSSFEKNPIDFLEAKGYKEVKKDLIIKLPKYSLFELENGRKR
MLASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDSGGSGGSGGSTNLSDIIEKETGK
QLVIQESILMLPEEVEEVIGNKPESDILVHTAYDESTDENVMLLTSDAPEYKPWALVIQD
SNGENKIKMLSGGSGGSGGSTNLSDIIEKETGKQLVIQESILMLPEEVEEVIGNKPESDIL
VHTAYDESTDENVMLLTSDAPEYKPWALVIQDSNGENKIKMLSGGSKRTADGSEFEPK
KKRKV
>SEQ ID NO: 77 Sdd7-BE4max 
MKRTADGSEFESPKKKRKVLEAVRARLIGEGGGPGAVPEGGDGPPAVPAEEVERL
RGELPPPVVPGTGQKTHGRWIGPDGRVRAIVSGRDEDAALVHAQLAAKGIPDEPTRNS
DVEQKLAAHMVANGIRHVTLVINHRPCRGFDDSCDTLVPIILPEGCTLTVHGQTDKGM
RVRVRYTGGARPWWSSGGSSGGSSGSETPGTSESATPESSGGSSGGSDKKYSIGLAIGT
NSVGWAVITDEYKVPSKKFKVLGNTDRHSIKKNLIGALLFDSGETAEATRLKRTARRRY
TRRKNRICYLQEIFSNEMAKVDDSFFHRLEESFLVEEDKKHERHPIFGNIVDEVAYHEK
YPTIYHLRKKLVDSTDKADLRLIYLALAHMIKFRGHFLIEGDLNPDNSDVDKLFIQLVQ
TYNQLFEENPINASGVDAKAILSARLSKSRRLENLIAQLPGEKKNGLFGNLIALSLGLTP
NFKSNFDLAEDAKLQLSKDTYDDDLDNLLAQIGDQYADLFLAAKNLSDAILLSDILRV
NTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQE
DFYPFLKDNREKIEKILTFRIPYYVGPLARGNSRFAWMTRKSEETITPWNFEEVVDKGA
SAQSFIERMTNFDKNLPNEKVLPKHSLLYEYFTVYNELTKVKYVTEGMRKPAFLSGEQ
KKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGR
LSRKLINGIRDKQSGKTILDFLKSDGFANRNFMQLIHDDSLTFKEDIQKAQVSGQGDSL
HEHIANLAGSPAIKKGILQTVKVVDELVKVMGRHKPENIVIEMARENQTTQKGQKNSR
 
ERMKRIEEGIKELGSQILKEHPVENTQLQNEKLYLYYLQNGRDMYVDQELDINRLSDY
DVDHIVPQSFLKDDSIDNKVLTRSDKNRGKSDNVPSEEVVKKMKNYWRQLLNAKLIT
QRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVY
GDYKVYDVRKMIAKSEQEIGKATAKYFFYSNIMNFFKTEITLANGEIRKRPLIETNGET
GEIVWDKGRDFATVRKVLSMPQVNIVKKTEVQTGGFSKESILPKRNSDKLIARKKDWD
PKKYGGFDSPTVAYSVLVVAKVEKGKSKKLKSVKELLGITIMERSSFEKNPIDFLEAKG
YKEVKKDLIIKLPKYSLFELENGRKRMLASAGELQKGNELALPSKYVNFLYLASHYEK
LKGSPEDNEQKQLFVEQHKHYLDEIIEQISEFSKRVILADANLDKVLSAYNKHRDKPIR
EQAENIIHLFTLTNLGAPAAFKYFDTTIDRKRYTSTKEVLDATLIHQSITGLYETRIDLSQ
LGGDSGGSGGSGGSTNLSDIIEKETGKQLVIQESILMLPEEVEEVIGNKPESDILVHTAYD
ESTDENVMLLTSDAPEYKPWALVIQDSNGENKIKMLSGGSGGSGGSTNLSDIIEKETGK
QLVIQESILMLPEEVEEVIGNKPESDILVHTAYDESTDENVMLLTSDAPEYKPWALVIQD
SNGENKIKMLSGGSKRTADGSEFEPKKKRKV
>SEQ ID NO: 78 mini-Sdd3-BE4max 
MKRTADGSEFESPKKKRKVPRRFNPACWQGLKPYDQAGTAEGNLFIAPGKRWNT
RPMQASKLEVGPQSDLHPQWRSRKAPWHIEGKIAAYMRQKGFTDGCVYLNARPCSGP
DGCARNLPDLLPVGSTLHVHARYIDRTGETRFYYREYRGTGKALTSGGSSGGSSGSETP
GTSESATPESSGGSSGGSDKKYSIGLAIGTNSVGWAVITDEYKVPSKKFKVLGNTDRHSI
KKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHM
IKFRGHFLIEGDLNPDNSDVDKLFIQLVQTYNQLFEENPINASGVDAKAILSARLSKSRR
LENLIAQLPGEKKNGLFGNLIALSLGLTPNFKSNFDLAEDAKLQLSKDTYDDDLDNLL
AQIGDQYADLFLAAKNLSDAILLSDILRVNTEITKAPLSASMIKRYDEHHQDLTLLKALV
RQQLPEKYKEIFFDQSKNGYAGYIDGGASQEEFYKFIKPILEKMDGTEELLVKLNREDL
LRKQRTFDNGSIPHQIHLGELHAILRRQEDFYPFLKDNREKIEKILTFRIPYYVGPLARGN
SRFAWMTRKSEETITPWNFEEVVDKGASAQSFIERMTNFDKNLPNEKVLPKHSLLYEY
FTVYNELTKVKYVTEGMRKPAFLSGEQKKAIVDLLFKTNRKVTVKQLKEDYFKKIECF
DSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNF
MQLIHDDSLTFKEDIQKAQVSGQGDSLHEHIANLAGSPAIKKGILQTVKVVDELVKVM
GRHKPENIVIEMARENQTTQKGQKNSRERMKRIEEGIKELGSQILKEHPVENTQLQNE
KLYLYYLQNGRDMYVDQELDINRLSDYDVDHIVPQSFLKDDSIDNKVLTRSDKNRGKS
DNVPSEEVVKKMKNYWRQLLNAKLITQRKFDNLTKAERGGLSELDKAGFIKRQLVET
 
RQITKHVAQILDSRMNTKYDENDKLIREVKVITLKSKLVSDFRKDFQFYKVREINNYHH
AHDAYLNAVVGTALIKKYPKLESEFVYGDYKVYDVRKMIAKSEQEIGKATAKYFFYSN
IMNFFKTEITLANGEIRKRPLIETNGETGEIVWDKGRDFATVRKVLSMPQVNIVKKTEV
QTGGFSKESILPKRNSDKLIARKKDWDPKKYGGFDSPTVAYSVLVVAKVEKGKSKKLK
SVKELLGITIMERSSFEKNPIDFLEAKGYKEVKKDLIIKLPKYSLFELENGRKRMLASAG
ELQKGNELALPSKYVNFLYLASHYEKLKGSPEDNEQKQLFVEQHKHYLDEIIEQISEFS
KRVILADANLDKVLSAYNKHRDKPIREQAENIIHLFTLTNLGAPAAFKYFDTTIDRKRYT
STKEVLDATLIHQSITGLYETRIDLSQLGGDSGGSGGSGGSTNLSDIIEKETGKQLVIQESI
LMLPEEVEEVIGNKPESDILVHTAYDESTDENVMLLTSDAPEYKPWALVIQDSNGENKI
KMLSGGSGGSGGSTNLSDIIEKETGKQLVIQESILMLPEEVEEVIGNKPESDILVHTAYDE
STDENVMLLTSDAPEYKPWALVIQDSNGENKIKMLSGGSKRTADGSEFEPKKKRKV
>SEQ ID NO: 79 Sdd7-QBEmax 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSLEAVRARLIGEGGGPGAVPEGGDGPP
AVPAEEVERLRGELPPPVVPGTGQKTHGRWIGPDGRVRAIVSGRDEDAALVHAQLAAK
GIPDEPTRNSDVEQKLAAHMVANGIRHVTLVINHRPCRGFDDSCDTLVPIILPEGCTLTV
HGQTDKGMRVRVRYTGGARPWWSSGGSSGGSSGSETPGTSESATPESSGGSSGGSLKG
SPEDNEQKQLFVEQHKHYLDEIIEQISEFSKRVILADANLDKVLSAYNKHRDKPIREQA
ENIIHLFTLTNLGAPAAFKYFDTTIDRKRYTSTKEVLDATLIHQSITGLYETRIDLSQLGG
DGGSGGSGGSGGSGGSGGSGGMDKKYSIGLAIGTNSVGWAVITDEYKVPSKKFKVLG
NTDRHSIKKNLIGALLFDSGETAEATRLKRTARRRYTRRKNRICYLQEIFSNEMAKVDD
SFFHRLEESFLVEEDKKHERHPIFGNIVDEVAYHEKYPTIYHLRKKLVDSTDKADLRLIY
LALAHMIKFRGHFLIEGDLNPDNSDVDKLFIQLVQTYNQLFEENPINASGVDAKAILSA
RLSKSRRLENLIAQLPGEKKNGLFGNLIALSLGLTPNFKSNFDLAEDAKLQLSKDTYDD
DLDNLLAQIGDQYADLFLAAKNLSDAILLSDILRVNTEITKAPLSASMIKRYDEHHQDL
TLLKALVRQQLPEKYKEIFFDQSKNGYAGYIDGGASQEEFYKFIKPILEKMDGTEELLV
KLNREDLLRKQRTFDNGSIPHQIHLGELHAILRRQEDFYPFLKDNREKIEKILTFRIPYYV
GPLARGNSRFAWMTRKSEETITPWNFEEVVDKGASAQSFIERMTNFDKNLPNEKVLPK
HSLLYEYFTVYNELTKVKYVTEGMRKPAFLSGEQKKAIVDLLFKTNRKVTVKQLKED
YFKKIECFDSVEISGVEDRFNASLGTYHDLLKIIKDKDFLDNEENEDILEDIVLTLTLFED
REMIEERLKTYAHLFDDKVMKQLKRRRYTGWGRLSRKLINGIRDKQSGKTILDFLKSD
 
GFANRNFMQLIHDDSLTFKEDIQKAQVSGQGDSLHEHIANLAGSPAIKKGILQTVKVVD
ELVKVMGRHKPENIVIEMARENQTTQKGQKNSRERMKRIEEGIKELGSQILKEHPVEN
TQLQNEKLYLYYLQNGRDMYVDQELDINRLSDYDVDHIVPQSFLKDDSIDNKVLTRSD
KNRGKSDNVPSEEVVKKMKNYWRQLLNAKLITQRKFDNLTKAERGGLSELDKAGFIK
RQLVETRQITKHVAQILDSRMNTKYDENDKLIREVKVITLKSKLVSDFRKDFQFYKVRE
INNYHHAHDAYLNAVVGTALIKKYPKLESEFVYGDYKVYDVRKMIAKSEQEIGSGGSG
GSGGSTNLSDIIEKETGKQLVIQESILMLPEEVEEVIGNKPESDILVHTAYDESTDENVML
LTSDAPEYKPWALVIQDSNGENKIKMLSGGSGGSGGSTNLSDIIEKETGKQLVIQESILM
LPEEVEEVIGNKPESDILVHTAYDESTDENVMLLTSDAPEYKPWALVIQDSNGENKIKM
LSGGSKRTADGSEFEPKKKRKV
>SEQ ID NO: 80 mini-Sdd9-QBEmax 
MKRTADGSEFESPKKKRKVKATAKYFFYSNIMNFFKTEITLANGEIRKRPLIETNG
ETGEIVWDKGRDFATVRKVLSMPQVNIVKKTEVQTGGFSKESILPKRNSDKLIARKKD
WDPKKYGGFDSPTVAYSVLVVAKVEKGKSKKLKSVKELLGITIMERSSFEKNPIDFLEA
KGYKEVKKDLIIKLPKYSLFELENGRKRMLASAGELQKGNELALPSKYVNFLYLASHY
EKSGGSSGGSSGSETPGTSESATPESSGGSSGGSCPIWVKNALQELVGRKETSGKVFDV
DGKPIGPDIQSGYKDRELRRGVYETLRKSPFFQKHFPSTATWYVSLHVEAQYAVWMLR
NRIKHATVVINNTYVCSDMNRLHDNCMTAVPHILPEGYTMTVWKADRTEVTLRGKAP
KESGGSSGGSSGSETPGTSESATPESSGGSSGGSLKGSPEDNEQKQLFVEQHKHYLDEII
EQISEFSKRVILADANLDKVLSAYNKHRDKPIREQAENIIHLFTLTNLGAPAAFKYFDTTI
DRKRYTSTKEVLDATLIHQSITGLYETRIDLSQLGGDGGSGGSGGSGGSGGSGGSGGM
DKKYSIGLAIGTNSVGWAVITDEYKVPSKKFKVLGNTDRHSIKKNLIGALLFDSGETAE
ATRLKRTARRRYTRRKNRICYLQEIFSNEMAKVDDSFFHRLEESFLVEEDKKHERHPIF
GNIVDEVAYHEKYPTIYHLRKKLVDSTDKADLRLIYLALAHMIKFRGHFLIEGDLNPDN
SDVDKLFIQLVQTYNQLFEENPINASGVDAKAILSARLSKSRRLENLIAQLPGEKKNGL
FGNLIALSLGLTPNFKSNFDLAEDAKLQLSKDTYDDDLDNLLAQIGDQYADLFLAAKN
LSDAILLSDILRVNTEITKAPLSASMIKRYDEHHQDLTLLKALVRQQLPEKYKEIFFDQS
KNGYAGYIDGGASQEEFYKFIKPILEKMDGTEELLVKLNREDLLRKQRTFDNGSIPHQI
HLGELHAILRRQEDFYPFLKDNREKIEKILTFRIPYYVGPLARGNSRFAWMTRKSEETIT
PWNFEEVVDKGASAQSFIERMTNFDKNLPNEKVLPKHSLLYEYFTVYNELTKVKYVTE
GMRKPAFLSGEQKKAIVDLLFKTNRKVTVKQLKEDYFKKIECFDSVEISGVEDRFNAS
LGTYHDLLKIIKDKDFLDNEENEDILEDIVLTLTLFEDREMIEERLKTYAHLFDDKVMK
QLKRRRYTGWGRLSRKLINGIRDKQSGKTILDFLKSDGFANRNFMQLIHDDSLTFKEDI
QKAQVSGQGDSLHEHIANLAGSPAIKKGILQTVKVVDELVKVMGRHKPENIVIEMARE
 
NQTTQKGQKNSRERMKRIEEGIKELGSQILKEHPVENTQLQNEKLYLYYLQNGRDMY
VDQELDINRLSDYDVDHIVPQSFLKDDSIDNKVLTRSDKNRGKSDNVPSEEVVKKMKN
YWRQLLNAKLITQRKFDNLTKAERGGLSELDKAGFIKRQLVETRQITKHVAQILDSRM
NTKYDENDKLIREVKVITLKSKLVSDFRKDFQFYKVREINNYHHAHDAYLNAVVGTAL
IKKYPKLESEFVYGDYKVYDVRKMIAKSEQEIGSGGSGGSGGSTNLSDIIEKETGKQLV
IQESILMLPEEVEEVIGNKPESDILVHTAYDESTDENVMLLTSDAPEYKPWALVIQDSNG
ENKIKMLSGGSGGSGGSTNLSDIIEKETGKQLVIQESILMLPEEVEEVIGNKPESDILVHT
AYDESTDENVMLLTSDAPEYKPWALVIQDSNGENKIKMLSGGSKRTADGSEFEPKKKR
KV
>SEQ ID NO: 81 Sdd2 
APDSLVWFDPLGLIVLQQVPYNDHPLFGAVSEFIQGKSRSDLRGRNVAAVLLDDG
TVIVRASEGGGNHAERVLMGLSEVDPAKVVAVYTERSPCTGRINCHDLLDSSLGADVP
VYYTHEMIRGQEGKTAQQIEADRNQFCRGG
>SEQ ID NO: 82 Sdd3 
SASAQLNTYLAAIGNSTTTVEAQPEAAPPPAAAESLDSTPRLPDGGIDFHALAKRL
GLLEARPTEQPPFDPRRFNPACWQGLKPYDQAGTAEGNLFIAPGKRWNTRPMQASKLE
VGPQSDLHPQWRSRKAPWHIEGKIAAYMRQKGFTDGCVYLNARPCSGPDGCARNLPD
LLPVGSTLHVHARYIDRTGETRFYYREYRGTGKALT
>SEQ ID NO: 83 Sdd4 
LDAMDAYLSEIAGGNAPARAGPKAPEPKQPGGSSSPRARDGRIDFRALLERLKAQ
GVVGLEGRSDDPIPDFDPKKQNPACYQGLAPRQKGKPVRGNLFFPDGRRWNDVALES
SRGEPAFDLNIIKPEYRSLSPARGHLEGNVAAWMRSTFHQEMVLYINESPCRKHGKGCL
YTLEHFLPRGYVLHVWSRNDRGEWRGNTFRGSGEAFTEGA
>SEQ ID NO: 84 Sdd6 
VETRDKIIAAKSRSDAGLLAFQQATNGSIDSRPAEAIANLQRAKTHLDEAQRLVAN
SDAAVDNYINAILGGASAATAQPSAVIPASKPSRFKPMRTDPAKADEIRPHVGKDRAVAT
LWDADGNRVLGLHSADDDGPAATAAWKPPWRDYVRLRRHVEAHAAARMHQDGHKT
MVMYINLPPCKYFDGCKLNLEDILPKGSTLWMHRVFQNGGTKIYQFNGTGRAYV
>SEQ ID NO: 85 Sdd7 LEAVRARLIGEGGGPGAVPEGGDGPPAVPAEEVERLRGELPPPVVPGTGQKTHGR 
WIGPDGRVRAIVSGRDEDAALVHAQLAAKGIPDEPTRNSDVEQKLAAHMVANGIRHV
TLVINHRPCRGFDDSCDTLVPIILPEGCTLTVHGQTDKGMRVRVRYTGGARPWWS
>SEQ ID NO: 86 Sdd9 
LDVDDPHTFYVLAGKTPVLVHNSECPIWVKNALQELVGRKETSGKVFDVDGKPI
GPDIQSGYKDRELRRGVYETLRKSPFFQKHFPSTATWYVSLHVEAQYAVWMLRNRIKH
ATVVINNTYVCSDMNRLHDNCMTAVPHILPEGYTMTVWKADRTEVTLRGKAPKE
>SEQ ID NO: 87 Sdd10 
LDAALGAVRRIIAALGTSGAERASPGANGSERVDELAERLPPTVVPNTSAKTHGW
WFTGQGAAQELISGEGPDARAAYEALREEGYPRPGMPFVAMHVEIKLAAHMRRNDIE
HATVVINNIPCPLVWGCENLIGVVLPEGSSLTVHGSNGYERTFTGGRKPPWPR
>SEQ ID NO: 88 Sdd59 
LLTPPPRPAAPPTTRPKPLVARTGDAYPPGTEWALPLIVQPHPPVGGTVPVEGHVRA
LRPESQISHVFHPGGGHWTEQARARLRVLPGFGWAVNLGHHVELQIAAWMTACGIHH
AELVLNRPPCGERYGLGCHQALPVLLPRGYRLTVSSTRGGPQPYQHHYEGKA
>SEQ ID NO: 89 UGI 
TNLSDIIEKETGKQLVIQESILMLPEEVEEVIGNKPESDILVHTAYDESTDENVMLLT
SDAPEYKPWALVIQDSNGENKIKML
>SEQ ID NO: 90 Linker
   GGSGGSGGSGGSGGSGGSGG
>SEQ ID NO: 91 Circularly Permuted Sequence 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPGEKK
NGLFGNLIALSLGLTPNFKSNFDLAEDAKLQLSKDTYDDDLDNLLAQIGDQYADLFLA
AKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIP
HQIHLGELHAILRRQEDFYPFLKDNREKIEKILTFRIPYYVGPLARGNSRFAWMTRKSEE
TITPWNFEEVVDKGASAQSFIERMTNFDKNLPNEKVLPKHSLLYEYFTVYNELTKVKY
 
VTEGMRKPAFLSGEQKKAIVDLLFKTNRKVTVKQLKEDYFKKIECFDSVEISGVEDRF
NASLGTYHDLLKIIKDKDFLDNEENEDILEDIVLTLTLFEDREMIEERLKTYAHLFDDKV
MKQLKRRRYTGWGRLSRKLINGIRDKQSGKTILDFLKSDGFANRNFMQLIHDDSLTFK
EDIQKAQVSGQGDSLHEHIANLAGSPAIKKGILQTVKVVDELVKVMGRHKPENIVIEM
ARENQTTQKGQKNSRERMKRIEEGIKELGSQILKEHPVENTQLQNEKLYLYYLQNGRD
MYVDQELDINRLSDYDVDHIVPQSFLKDDSIDNKVLTRSDKNRGKSDNVPSEEVVKK
MKNYWRQLLNAKLITQRKFDNLTKAERGGLSELDKAGFIKRQLVETRQITKHVAQILD
SRMNTKYDENDKLIREVKVITLKSKLVSDFRKDFQFYKVREINNYHHAHDAYLNAVV
GTALIKKYPKLESEFVYGDYKVYDVRKMIAKSEQEIGKATAKYFFYSNIMNFFKTEITL
ANGEIRKRPLIETNGETGEIVWDKGRDFATVRKVLSMPQVNIVKKTEVQTGGFSKESIL
PKRNSDKLIARKKDWDPKKYGGFDSPTVAYSVLVVAKVEKGKSKKLKSVKELLGITIM
ERSSFEKNPIDFLEAKGYKEVKKDLIIKLPKYSLFELENGRKRMLASAGELQKGNELAL
PSKYVNFLYLASHYEKLKGSPEDNEQKQLFVEQHKHYLDEIIEQISEFSKRVILADANL
DKVLSAYNKHRDKPIREQAENIIHLFTLTNLGAPAAFKYFDTTIDRKRYTSTKEVLDATL
IHQSITGLYETRIDLSQLGGDGGSGGSGGSGGSGGSGG
>SEQ ID NO: 92 QBE3-N 
KATAKYFFYSNIMNFFKTEITLANGEIRKRPLIETNGETGEIVWDKGRDFATVRKV
LSMPQVNIVKKTEVQTGGFSKESILPKRNSDKLIARKKDWDPKKYGGFDSPTVAYSVL
VVAKVEKGKSKKLKSVKELLGITIMERSSFEKNPIDFLEAKGYKEVKKDLIIKLPKYSLF
ELENGRKRMLASAGELQKGNELALPSKYVNFLYLASHYEK
>SEQ ID NO: 93 QBE3-C 
LKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGDGGSGGSGGSGGSGGSGGSGGMDKKYSIGLAIGTNSVGWAVITDEYKVPSKK
FKVLGNTDRHSIKKNLIGALLFDSGETAEATRLKRTARRRYTRRKNRICYLQEIFSNEM
AKVDDSFFHRLEESFLVEEDKKHERHPIFGNIVDEVAYHEKYPTIYHLRKKLVDSTDKA
DLRLIYLALAHMIKFRGHFLIEGDLNPDNSDVDKLFIQLVQTYNQLFEENPINASGVDA
KAILSARLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMIKRYDE
HHQDLTLLKALVRQQLPEKYKEIFFDQSKNGYAGYIDGGASQEEFYKFIKPILEKMDGT
EELLVKLNREDLLRKQRTFDNGSIPHQIHLGELHAILRRQEDFYPFLKDNREKIEKILTFR
IPYYVGPLARGNSRFAWMTRKSEETITPWNFEEVVDKGASAQSFIERMTNFDKNLPNE
KVLPKHSLLYEYFTVYNELTKVKYVTEGMRKPAFLSGEQKKAIVDLLFKTNRKVTVK
 
QLKEDYFKKIECFDSVEISGVEDRFNASLGTYHDLLKIIKDKDFLDNEENEDILEDIVLTL
TLFEDREMIEERLKTYAHLFDDKVMKQLKRRRYTGWGRLSRKLINGIRDKQSGKTILD
FLKSDGFANRNFMQLIHDDSLTFKEDIQKAQVSGQGDSLHEHIANLAGSPAIKKGILQT
VKVVDELVKVMGRHKPENIVIEMARENQTTQKGQKNSRERMKRIEEGIKELGSQILKE
HPVENTQLQNEKLYLYYLQNGRDMYVDQELDINRLSDYDVDHIVPQSFLKDDSIDNK
VLTRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDFQ
FYKVREINNYHHAHDAYLNAVVGTALIKKYPKLESEFVYGDYKVYDVRKMIAKSEQEI
G
>SEQ ID No.94 QBE3-X₂ 
KATAKYFFYSNIMNFFKTEITLANGEIRKRPLIETNGETGEIVWDKGRDFATVRKV
LSMPQVNIVKKTEVQTGGFSKESILPKRNSDKLIARKKDWDPKKYGGFDSPTVAYSVL
VVAKVEKGKSKKLKSVKELLGITIMERSSFEKNPIDFLEAKGYKEVKKDLIIKLPKYSLF
ELENGRKRMLASAGELQKGNELALPSKYVNFLYLASHYEK
>SEQ ID No.95 QBE3-X₃ 
LKGSPEDNEQKQLFVEQHKHYLDEIIEQISEFSKRVILADANLDKVLSAYNKHRDK
PIREQAENIIHLFTLTNLGAPAAFKYFDTTIDRKRYTSTKEVLDATLIHQSITGLYETRIDL
SQLGGD
>SEQ ID No.96 QBE3-X₄ 
MDKKYSIGLAIGTNSVGWAVITDEYKVPSKKFKVLGNTDRHSIKKNLIGALLFDSG
ETAEATRLKRTARRRYTRRKNRICYLQEIFSNEMAKVDDSFFHRLEESFLVEEDKKHER
HPIFGNIVDEVAYHEKYPTIYHLRKKLVDSTDKADLRLIYLALAHMIKFRGHFLIEGDLN
PDNSDVDKLFIQLVQTYNQLFEENPINASGVDAKAILSARLSKSRRLENLIAQLPGEKK
NGLFGNLIALSLGLTPNFKSNFDLAEDAKLQLSKDTYDDDLDNLLAQIGDQYADLFLA
AKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIP
HQIHLGELHAILRRQEDFYPFLKDNREKIEKILTFRIPYYVGPLARGNSRFAWMTRKSEE
TITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRF
NASLGTYHDLLKIIKDKDFLDNEENEDILEDIVLTLTLFEDREMIEERLKTYAHLFDDKV
MKQLKRRRYTGWGRLSRKLINGIRDKQSGKTILDFLKSDGFANRNFMQLIHDDSLTFK
EDIQKAQVSGQGDSLHEHIANLAGSPAIKKGILQTVKVVDELVKVMGRHKPENIVIEM
 
ARENQTTQKGQKNSRERMKRIEEGIKELGSQILKEHPVENTQLQNEKLYLYYLQNGRD
MYVDQELDINRLSDYDVDHIVPQSFLKDDSIDNKVLTRSDKNRGKSDNVPSEEVVKK
MKNYWRQLLNAKLITQRKFDNLTKAERGGLSELDKAGFIKRQLVETRQITKHVAQILD
SRMNTKYDENDKLIREVKVITLKSKLVSDFRKDFQFYKVREINNYHHAHDAYLNAVV
GTALIKKYPKLESEFVYGDYKVYDVRKMIAKSEQEIG
>SEQ ID NO: 97 Scaffold Sequence of sgRNA
   guuuuagagcuagaaauagcaaguuaaaauaaggcuaguccguuaucaacuugaaaaaguggcaccgagucggugc
> SEQ ID NO: 98 Scaffold Sequence of gRNA 
GUUUGAGAGCUAUGCUGGAAACAGCAUAGCAAGUUCAAAUAAGGCUAGUC
CGUUAUCAACUUGAAAAAGUGGCACCGAGUCGGUGC
> SEQ ID NO: 99 Scaffold Sequence of gRNA

GUUUUAGAGCUAGAAAUAGCAAGUUAAAAUAAGGCUAGUCCGUAAUCAAC
UUUUAAAGAGUGAGCACUGAUUCGGUGC

## Claims

1. A method for obtaining a modified T-cell, wherein the method comprises knocking out or downregulating expression of an endogenous gene in a T-cell by a gene editing technology, wherein the endogenous gene comprises a T-cell receptor alpha chain constant (TRAC) gene, a transforming growth factor beta receptor 2 (TGFBR2) gene, a programmed death receptor 1 (PD-1) gene, a cytokine-inducible SH2-containing protein (CISH) gene, and a cell suicide-associated factor (FAS) gene.

2. The method according to claim 1, wherein the gene editing technology comprises DNA insertion, deletion or substitution at a gene target, and preferably, the gene editing technology is DNA substitution at a gene target.

3. The method according to claim 1, wherein the gene editing technology comprises single-base editing.

4. The method according to any one of claims 1 to 3, wherein the method comprises:
a) providing a purified T-cell population;
b) introducing a single-base editing system into the T-cell population of a); and
c) amplifying and harvesting the modified T-cell;
wherein the single-base editing system in step b) comprises: i) a base editing polypeptide, and/or an expression construct encoding the base editing polypeptide; and ii) a guide RNA (gRNA) containing a spacer corresponding to a protospacer in the *TRAC*, *TGFBR2*, *PD-1*, *CISH* or *FAS* gene, and/or an expression construct encoding the guide RNA.

5. The method according to claim 4, wherein a protospacer sequence of the *TRAC* gene comprises SEQ ID NO: 51; a protospacer sequence of the *TGFBR2* gene comprises SEQ ID NO: 21, SEQ ID NO: 24, SEQ ID NO: 27, or SEQ ID NO: 28; a protospacer sequence of the *PD-1* gene comprises SEQ ID NO: 41, SEQ ID NO: 44, SEQ ID NO: 45, SEQ ID NO: 46, or SEQ ID NO: 47; a protospacer sequence of the *CISH* gene comprises SEQ ID NO: 1, SEQ ID NO: 5, SEQ ID NO: 8, or SEQ ID NO: 10; and a protospacer sequence of the *FAS* gene comprises SEQ ID NO: 14, SEQ ID NO: 17, SEQ ID NO: 18, or SEQ ID NO: 20.

6. The method according to claim 4, wherein a protospacer sequence of the *TRAC* gene comprises SEQ ID NO: 51; a protospacer sequence of the *TGFBR2* gene comprises SEQ ID NO: 24 or SEQ ID NO: 28; a protospacer sequence of the *PD-1* gene comprises SEQ ID NO: 41 or SEQ ID NO: 47; a protospacer sequence of the *CISH* gene comprises SEQ ID NO: 1; and a protospacer sequence of the *FAS* gene comprises SEQ ID NO: 14.

7. The method according to any one of claims 4 to 6, wherein the base editing polypeptide comprises a DNA-binding protein and at least one deaminase domain.

8. The method according to any one of claims 4 to 7, wherein the base editing polypeptide further comprises at least one uracil DNA glycosylase inhibitor (UGI) domain.

9. The method according to any one of claims 4 to 8, wherein the base editing polypeptide further comprises a nuclear localization sequence.

10. The method according to any one of claims 4 to 9, wherein the deaminase is a cytidine deaminase; optionally, the cytidine deaminase is an SCP1.201 family deaminase; alternatively, the SCP1.201 family deaminase is an Sdd2 deaminase, an Sdd3 deaminase, an Sdd4 deaminase, an Sdd6 deaminase, an Sdd7 deaminase, a mini-Sdd7 deaminase, a mini-Sdd9 deaminase, an Sdd10 deaminase, an Sdd59 deaminase, a mini-Sdd3 deaminase, or a mini-Sdd6 deaminase.

11. The method according to any one of claims 4 to 10, wherein the deaminase is Sdd7 and/or mini-Sdd9 cytidine deaminase(s).

12. The method according to any one of claims 4 to 11, wherein the DNA-binding protein is a TALE, ZFP or CRISPR effector protein or a variant thereof; optionally, the CRISPR effector protein is a Cas9 nickase, a deactivated Cas9, or a variant thereof.

13. The method according to any one of claims 4 to 12, wherein the base editing polypeptide comprises an amino acid sequence having at least 80%, 85%, 90%, 95%, 96%, 97%, 98%, or 99% sequence identity to any one of SEQ ID NOs: 74-80.

14. The method according to any one of claims 4 to 13, wherein the method further comprises introducing a nucleic acid sequence encoding a chimeric antigen receptor (CAR), wherein the chimeric antigen receptor (CAR) comprises an antigen-binding domain and a transmembrane domain.

15. The method according to any one of claims 4 to 14, wherein the T-cell population is T-cells derived from humans, optionally T-cells enriched from human blood cells, and alternatively cryopreserved T-cells enriched from human blood cells.

16. The method according to any one of claims 4 to 15, wherein a method for the introduction comprises electroporation, liposome transfection, microinjection, viral infection (such as adenovirus, adeno-associated virus, lentivirus, or other viruses), N-acetylgalactosamine (GalNAc)-mediated transformation, gene gun-mediated transformation, ribonucleoprotein (RNP) complex-mediated transformation, and nanoparticle-mediated transformation.

17. A base editing system of modifying a T-cell, comprising:
i) a base editing polypeptide, and/or an expression construct encoding the base editing polypeptide, wherein the base editing polypeptide comprises a DNA-binding protein and at least one deaminase domain; and
ii) a guide RNA, and/or an expression construct encoding the guide RNA, wherein the guide RNA targets any one or any combination of a T-cell receptor alpha chain constant (TRAC) gene, a transforming growth factor beta receptor 2 (TGFBR2) gene, a programmed death receptor 1 (PD-1) gene, a cytokine-inducible SH2-containing protein (CISH) gene, and/or a cell suicide-associated factor (FAS) genomic region.

18. The base editing system according to claim 17, wherein the deaminase domain is a cytidine deaminase.

19. The base editing system according to claim 18, wherein the cytidine deaminase is an APOBEC family deaminase, an SCP1.201 family deaminase or a homolog thereof;
optionally, the APOBEC family deaminase is an AID deaminase, an APOBEC1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, or an APOBEC3H deaminase; and
optionally, the SCP1.201 family deaminase is an Sdd2 deaminase, an Sdd3 deaminase, an Sdd4 deaminase, an Sdd6 deaminase, an Sdd7 deaminase, a mini-Sdd7 deaminase, a mini-Sdd9 deaminase, an Sdd10 deaminase, an Sdd59 deaminase, a mini-Sdd3 deaminase, or a mini-Sdd6 deaminase.

20. The base editing system according to claim 19, wherein the cytidine deaminase is Sdd7 and/or mini-Sdd9 cytidine deaminase(s).

21. The base editing system according to any one of claims 17 to 20, wherein the DNA-binding protein is a TALE, ZFP or CRISPR effector protein.

22. The base editing system according to claim 21, wherein the CRISPR effector protein is Cas3, Cas4, Cas5, Cas5e (or CasD), Cas6, Cas6e, Cas6f, Cas7, Cas8a1, Cas8a2, Cas8b, Cas8c, Cas9, Cas10, Cast10d, Cas12, Cas13, Cas14, CasX, CasF, CasG, CasH, Csy1, Csy2, Csy3, Cse1 (or CasA), Cse2 (or CasB), Cse3 (or CasE), Cse4 (or CasC), Csc1, Csc2, Csa5, Csn1, Csn2, Csm2, Csm3, Csm4, Csm5, Csm6, Cmrl, Cmr3, Cmr4, Cmr5, Cmr6, Cpf1, Csb1, Csb2, Csb3, Csx17, Csx14, Csx10, Csx16, CsaX, Csx3, Csz1, Csx15, Csf1, Csf2, Csf3, Csf4, Cu1966, TraC, or a variant thereof.

23. The base editing system according to claim 22, wherein the DNA-binding protein is a Cas9 nickase, a deactivated Cas9 or a variant thereof.

24. The base editing system according to any one of claims 17 to 23, wherein the base editing system further contains at least one uracil DNA glycosylase inhibitor (UGI).

25. The base editing system according to any one of claims 17 to 24, wherein the base editing system further comprises a nuclear localization sequence.

26. The base editing system according to any one of claims 17 to 25, wherein the base editing polypeptide comprises an amino acid sequence having at least 80% sequence identity to any one of SEQ ID NOs: 74-80.

27. The base editing system according to claim 26, wherein the base editing polypeptide comprises an amino acid sequence having at least 80%, 85%, 90%, 95%, 96%, 97%, 98%, or 99% sequence identity to any one of SEQ ID NOs: 77-80.

28. The base editing system according to any one of claims 17 to 27, wherein the guide RNA contains a spacer corresponding to a protospacer in the *TRAC*, *TGFBR2*, *PD-1*, *CISH*, or *FAS* gene;
optionally, a protospacer sequence of the *TRAC* gene comprises SEQ ID NO: 51; a protospacer sequence of the *TGFBR2* gene comprises SEQ ID NO: 21, SEQ ID NO: 24, SEQ ID NO: 27, or SEQ ID NO: 28; a protospacer sequence of the *PD-1* gene comprises SEQ ID NO: 41, SEQ ID NO: 44, SEQ ID NO: 45, SEQ ID NO: 46, or SEQ ID NO: 47; a protospacer sequence of the *CISH* gene comprises SEQ ID NO: 1, SEQ ID NO: 5, SEQ ID NO: 8, or SEQ ID NO: 10; and a protospacer sequence of the *FAS* gene comprises SEQ ID NO: 14, SEQ ID NO: 17, SEQ ID NO: 18, or SEQ ID NO: 20.

29. The base editing system according to claim 28, wherein the protospacer sequence of the *TRAC* gene comprises SEQ ID NO: 51; the protospacer sequence of the *TGFBR2* gene comprises SEQ ID NO: 24 or SEQ ID NO: 28; the protospacer sequence of the *PD-1* gene comprises SEQ ID NO: 41 or SEQ ID NO: 47; the protospacer sequence of the *CISH* gene comprises SEQ ID NO: 1; and the protospacer sequence of the *FAS* gene comprises SEQ ID NO: 14.

30. A modified T-cell, wherein the T-cell is modified by the method according to any one of claims 1 to 16, or introduced with the base editing system according to any one of claims 17 to 29, and the T-cell has an endogenous gene knocked out or downregulated in expression, wherein the endogenous gene comprises a T-cell receptor alpha chain constant (TRAC) gene, a transforming growth factor beta receptor 2 (TGFBR2) gene, a programmed death receptor 1 (PD-1) gene, a cytokine-inducible SH2-containing protein (CISH) gene, and a cell suicide-associated factor (FAS) gene.

31. The T-cell according to claim 30, wherein the T-cell is a CAR-T-cell.

32. The T-cell according to claim 31, wherein the CAR-T-cell recognizes one or more target antigens.

33. The T-cell according to any one of claims 30 to 32, wherein the T-cell is used for preventing or treating an autoimmune disease, a tumor, a viral infectious disease, a bacterial infectious disease, a genetic disease, an infectious disease, or a cardiovascular disease.

34. A composition, wherein the composition comprises the modified T-cell according to any one of claims 30 to 33, and optionally a pharmaceutically acceptable carrier or excipient.
